# EUROPÄISCHE PATENTSCHRIFT

(11) **EP 1 704 886 B1**
(45) Veröffentlichungstag und Bekanntmachung des Hinweises auf die Patenterteilung: **06.02.2008**
(21) Anmeldenummer: 05006545.7
(22) Anmeldetag: 24.03.2005
(51) Int. Cl.: A61M 5/142, A61M 5/145

(54) **Vorrichtung für die dosierte Verabreichung eines fluiden Produkts**
Device for the dosed administration of a fluid product
Dispositif pour la distribution dosée d'un produit fluide

(43) Veröffentlichungstag der Anmeldung: 27.09.2006
(73) Patentinhaber: Disetronic Licensing AG, 3401 Burgdorf (CH)
(72) Erfinder: Geiser, Simone, 4900 Langenthal (CH); Peter, Daniel, 3172 Niederwangen (CH)
(74) Vertreter: Wess, Wolfgang

(56) Entgegenhaltungen:
- EP-A- 0 514 816
- EP-A- 1 498 150
- WO-A-01/72357
- WO-A-20/04089448
- DE-A1- 19 717 107
- DE-C1- 4 133 402
- US-A- 5 492 534
- US-A1- 2004 122 366

## Beschreibung

Die Erfindung betrifft Vorrichtungen für die dosierte Verabreichung von fluiden Produkten in biotechnologischen Anwendungen, vorzugsweise in medizinischen einschließlich tiermedizinischen und pharmazeutischen Anwendungen. Insbesondere betrifft sie Infusionsgeräte und Injektionsgeräte.

In Therapien kommt der Genauigkeit der Dosierung der verabreichten Produkte eine große Bedeutung zu, beispielsweise der Verabreichung von Insulin in der Diabetestherapie. Gebräuchlich sind Infusionsgeräte und Injektionsgeräte, die ein zu verabreichendes Produkt mittels einer motorisch betriebenen Hubkolbenpumpe im Falle von Infusionsgeräten oder mittels einer manuell betätigten Hubkolbenpumpe im Falle von Injektionsgeräten aus einem Produktreservoir ausschütten. Bei Infusionsgeräten wird der Hubkolben üblicherweise von einem Rotationsantrieb angetrieben, wobei die Rotationsbewegung des Antriebs mittels eines Spindeltriebs in die Linearbewegung des Kolbens übertragen wird. Bei Injektionsgeräten dient ein Spindeltrieb häufig der Auswahl der zu verabreichenden Produktdosis, während die Linearbewegung des Kolbens unmittelbar manuell bewirkt wird. Bei Injektionsgeräten sind ferner Zahnstangengetriebe gebräuchlich. Den genannten Beispielen von Verabreichungsgeräten ist gemein, dass die Dosiergenauigkeit maßgeblich davon abhängt, wie genau die Weglänge vorgegeben werden kann, die der Kolben für die Förderung einer bestimmten Produktdosis zurücklegen muss.

Infusionsgeräte und Injektionsgeräte der vorstehend beschriebenen Art werden beispielsweise durch die DE 198 40 992 A, die DE 198 22 031 C und die DE 199 00 827 C beschrieben.

Besondere Anforderungen an die Dosiergenauigkeit und -feinheit stellen Infusionsgeräte, mit denen das Produkt oft über längere Zeiträume in kleinen, diskreten Boli abgegeben wird. Dabei kann es vorkommen, dass konstruktive Merkmale, die im Grunde der Verbesserung der Dosiergenauigkeit dienen, gleichzeitig auch einen störenden Einfluss ausüben können, so beispielsweise die Fähigkeit der Okklusionserkennung. Ein Infusionsgerät mit einer vorteilhaft gestalteten, automatischen Okklusionserkennung wird in der DE 198 40 992 beschrieben, die diesbezüglich hiermit auch für die Zwecke der Erfindung in Bezug genommen wird. Ein weiteres Gerät mit Okklusionserkennung beschreibt die WO 01/72357 A2. Für die Okklusionserkennung wird die Fördereinrichtung im Ganzen über einen Sensor an dem Gehäuse des Infusionsgeräts abgestützt. Um zu verhindern, dass durch diese Art der Abstützung Relativbewegungen zwischen der Fördereinrichtung und dem Produktbehältnis ermöglicht werden, schlägt die WO 01/72357 A2 für den Zusammenbau des Geräts vor, die gesamte Fördereinrichtung in Vorschubrichtung des Kolbens zunächst bis gegen einen von dem Gehäuse gebildeten Anschlag zu drücken, anschließend die Fördereinrichtung im Wesentlichen von dem Druck zu entlasten und schließlich eine Abschlusskappe in eine rückwärtige Gehäuseöffnung einzusetzen und mit dem Gehäuse zu verkleben. Die Kappe soll die Fördereinrichtung auf Anschlag gegen das Gehäuse halten. Als alternative Ausführung wird ferner vorgeschlagen, die Fördereinrichtung an ihrer von dem Kolben abgewandten Seite mittels eines elastischen Dichtrings an dem rückwärtigen Boden des Gehäuses abzustützen und einen zwischen der Rückseite der Fördereinrichtung und dem Gehäuseboden verbleibenden Hohlraum mit einem Füllmaterial auszugießen, beispielsweise mit Silikon. Das Füllmaterial soll im Wesentlichen inkompressibel sein, um den Sensor nicht zu entlasten.

Es ist eine Aufgabe der Erfindung, mit Vorrichtungen für die dosierte Verabreichung fluider Produkte die gewünschte Produktdosis genauer als bisher zu fördern.

Eine Vorrichtung für die dosierte Verabreichung eines fluiden Produkts, wie die Erfindung sie betrifft, umfasst ein Gehäuse, ein Reservoir für das Produkt und eine Fördereinrichtung. Das Gehäuse kann das Reservoir unmittelbar selbst bilden. Vorzugsweise bildet jedoch ein Behältnis, beispielsweise eine Ampulle, das Reservoir. Für solch ein Behältnis bildet das Gehäuse eine Aufnahme, um das Behältnis in definierter Lage zu halten. Vorzugsweise ist solch ein Behältnis in das Gehäuse eingesetzt. Das Behältnis kann, wie bereits heute bei Ampullen üblich, fertig konfektioniert sein, indem es mit einer definierten Produktmenge gefüllt ist und auch bereits ein das Behältnis rückwärtig abdichtender Kolben in dem Behältnis aufgenommen ist. Fertig konfektionierte Ampullen dieser Art sind für die Selbstverabreichung von Insulin in der Diabetestherapie üblich. Bei dem Produkt kann es sich um das bereits genannte Insulin, ein Wachstumshormon und grundsätzlich jedes andere medizinisch oder beispielsweise kosmetisch wirksame Produkt handeln. Die erfindungsgemäße Vorrichtung ist bevorzugt für die Selbstverabreichung konzipiert.

Die Fördereinrichtung umfasst wenigstens ein Rotationsglied und wenigstens ein Translationsglied. Das wenigstens eine Rotationsglied führt für die Förderung des Produkts relativ zu dem Gehäuse eine Rotationsbewegung um eine Rotationsachse aus. Das wenigstens eine Translationsglied führt für die Förderung des Produkts eine Translationsbewegung relativ zu dem Reservoir aus und ist zumindest während der Ausführung der Translationsbewegung mit dem Rotationsglied mechanisch so gekoppelt, dass die Rotationsbewegung die Translationsbewegung oder die Translationsbewegung die Rotationsbewegung bewirkt. Das Rotationsglied und das Translationsglied sind ferner zumindest während der Ausführung der Translationsbewegung mechanisch so miteinander gekoppelt, dass das Rotationsglied das Translationsglied gegen die Richtung der Translationsbewegung abstützt, was jedoch den Fall mit einschließen soll, dass das Rotationsglied und das Translationsglied eine Translationsbewegung gemeinsam ausführen. Der Translationsbewegung des Translationsglieds kann eine Rotationsbewegung überlagert sein, vorzugsweise führt das Translationsglied jedoch nur die Translationsbewegung aus. Die Translationsbewegung ist vorzugsweise eine Linearbewegung.

Vorzugsweise wird das Rotationsglied motorisch angetrieben, falls die Verabreichungsvorrichtung ein Infusionsgerät ist, wie dies bevorzugt wird. Handelt es sich bei der Verabreichungsvorrichtung um ein Injektionsgerät, so wird mit dem Rotationsglied vorzugsweise die zu verabreichende Produktdosis ausgewählt. Diese Auswahl-Rotationsbewegung wird vorzugsweise manuell bewirkt.

Der Begriff "axial" bezeichnet in allen Ausführungen der Erfindung die Rotationsachse des Rotationsglieds. Vorzugsweise hat die Rotationsachse die Translationsrichtung nicht nur als eine Richtungskomponente, sondern weist in Translationsrichtung und ist im Falle einer axialen Linearbewegung des Translationsglieds mit der Translationsachse dieser Bewegung identisch.

Die Vorrichtung umfasst ferner eine Drehlagerung für das Rotationsglied. Die Drehlagerung umfasst einen ein- oder mehrteiligen Lagerkörper, der das Rotationsglied um die Rotationsachse drehbar lagert und an dem das Rotationsglied axial abgestützt ist. Der Lagerkörper kann von dem Gehäuse gebildet werden oder ist ein von dem Gehäuse separater Körper, der an dem Gehäuse axial abgestützt ist. Handelt es sich um einen separaten Lagerkörper, so sollte das Gehäuse in einfachen Ausführungen den Lagerkörper so lagern, dass er sich axial relativ zu dem Gehäuse nicht bewegen kann. In ebenfalls bevorzugten Ausführungen ist der Lagerkörper allerdings relativ zu dem Gehäuse axial bewegbar. Das Gehäuse lagert in solchen Ausführungen den Lagerkörper und damit zusammen die Fördereinrichtung axial schwimmend. Im Falle eines relativ zu dem Gehäuse axial beweglichen Lagerkörpers sollten Maßnahmen getroffen werden, die ein Axialspiel zwischen dem Lagerkörper und dem Gehäuse verringern, vorzugsweise gar nicht erst entstehen lassen.

Die Drehlagerung des Rotationsglieds weist wenigstens vier Stützflächen auf, um das Rotationsglied axial an dem Lagerkörper abzustützen. Eine erste und eine zweite der Stützflächen sind mit dem Lagerkörper axial steif verbunden. Eine dritte und eine vierte der Stützflächen sind mit dem Rotationsglied axial steif verbunden. Von diesen Stützflächen sind entweder die axial steif mit dem Lagerkörper oder die axial steif mit dem Rotationskörper verbundenen Stützflächen axial voneinander abgewandt, und die jeweils anderen Stützflächen weisen axial aufeinander zu. Vorzugsweise weisen die axial steif mit dem Lagerkörper verbundenen Stützflächen axial aufeinander zu. Ferner weisen die erste Stützfläche und die dritte Stützfläche axial aufeinander zu, und es weisen die zweite Stützfläche und die vierte Stützfläche axial aufeinander zu. Als axial einander zugewandt werden je solche Paare von Stützflächen bezeichnet, deren Flächennormalen in axial entgegengesetzte Richtungen aufeinander zu weisen. Die Flächen des jeweiligen Flächenpaars müssen axial nicht fluchten. Sie müssen einander auch nicht unmittelbar gegenüberliegen. Mit der Spezifizierung, dass eine Stützfläche axial steif mit dem Lagerkörper oder dem Rotationsglied verbunden ist, soll ausgesagt werden, dass die jeweilige Stützfläche entweder unmittelbar von dem Lagerkörper oder dem Rotationsglied gebildet wird, d. h. in einem Stück, oder aber, dass ein weiterer Körper die betreffende Stützfläche bildet und dieser weitere Körper axial nicht bewegbar mit entweder dem Lagerkörper oder dem Rotationsglied verbunden ist, vorzugsweise vollkommen steif. Soweit Axialkräfte zwischen dem Rotationsglied und dem Lagerkörper auftreten, werden diese Axialkräfte über die genannten Stützflächen von dem Rotationsglied auf den Lagerkörper oder von dem Lagerkörper auf das Rotationsglied übertragen. In diesem Sinne ist das Rotationsglied über die Stützflächen an dem Lagerkörper axial abgestützt.

Die Drehlagerung umfasst nach der Erfindung eine Spielreduziereinrichtung. Die Spielreduziereinrichtung spannt wenigstens zwei der Stützflächen, die einander axial zugewandt sind, mit einer Presskraft axial aufeinander zu, so dass ein Axialspiel der Drehlagerung eliminiert oder zumindest reduziert wird. Die axiale Presskraft soll einerseits zwar ausreichen, um das Axialspiel der Drehlagerung zu eliminieren oder wenigstens zu reduzieren, die Presskraft sollte andererseits jedoch so klein wie möglich sein, da sie zur Vergrößerung der Lagerreibung beiträgt. Die beiden anderen Stützflächen, die einander axial zugewandt sind, werden durch die Presskraft axial entweder voneinander weg oder ebenfalls aufeinander zu gespannt. Dies hängt von dem Ort ab, an dem die Presskraft in die Drehlagerung eingebracht wird. So kann die Presskraft zwischen zwei axial einander zugewandten Stützflächen wirken, indem sie die beiden Stützflächen voneinander weg und die beiden anderen Stützflächen aufeinander zu spannt. Die Presskraft kann auch von außen aufgebracht werden und in diesem Fall beide Stützflächenpaare, die axial einander zugewandt sind, axial aufeinander zu spannen.

In einer bevorzugten Ausführung erzeugt die Spielreduziereinrichtung die Presskraft als Elastizitätskraft. Obgleich die Elastizitätskraft beispielsweise pneumatisch erzeugt werden kann, umfasst die Spielreduzierung vorzugsweise eine die Presskraft erzeugende mechanische Feder. Die Elastizität der Feder kann auf Materialelastizität beruhen, beispielsweise durch Verwendung einer Elastomerfeder. Bevorzugt ist die Elastizität jedoch eine Formelastizität. Die Feder kann beispielsweise eine auf Druck beanspruchte Spiralfeder sein. Bevorzugt wirkt die Feder jedoch in der Art einer Blattfeder, um axiale Länge zu sparen. Die Feder kann insbesondere als axial elastischer Ringkörper gebildet sein, der das Rotationsglied umgibt. Falls die Feder, wie bevorzugt, als Blattfeder gebildet ist oder in der Art einer Blattfeder wirkt, kann solch ein Ringkörper umlaufend gewellt sein, so dass die Elastizitätskraft durch eine axiale Stauchung der Wellenform des Ringkörpers erzeugt wird. Die Feder ist vorzugsweise mit einer axialen Vorspannkraft eingebaut. Die axiale Vorspannkraft sollte so gering als möglich sein, um die Lagerreibung gering zu halten. Sie sollte jedoch größer sein als eine Axialkraft, die im praktischen Gebrauch bei einem Auftreten eines sogenannten Syphoning-Effekts auf die Fördereinrichtung wirken kann. Ein Syphoning kann dann auftreten, wenn Produkt aufgrund seines Eigengewichts, d.h. aufgrund der Schwerkraft, aus dem Reservoir ausläuft und die Fördereinrichtung dem auslaufenden Produkt nachgesogen wird. Allerdings kann das Auftreten von Syphoning durch den Einbau eines Überdruckventils an oder stromabwärts von einem Auslass des Reservoirs verhindert werden. Ferner sollte die Federkraft verhindern, dass das Translationsglied zusammen mit dem Rotationsglied von einer Stützfläche des Lagerkörpers, gegen die das Rotationsglied von der Feder gespannt wird, unter den zu berücksichtigenden Gebrauchsbedingungen abheben kann. Die Federkraft sollte wenigstens das Eigengewicht von Translations- und Rotationsglied kompensieren. Bei Vorrichtungen, die das genannte Überdruckventil nicht aufweisen, sollte sie zusätzlich auch noch die von einem Syphoning-Effekt ausgehende Saugkraft kompensieren.

Die Presskraft kann in Translationsrichtung des Translationsglieds auf das Rotationsglied wirken, falls nämlich das Rotationsglied in Translationsrichtung auf Anschlag gegen eine der Stützflächen des Lagerkörpers ist. Die Presskraft kann jedoch auch stattdessen entgegen der Translationsrichtung auf das Rotationsglied wirken und das Rotationsglied entgegen der Translationsrichtung auf Anschlag gegen eine der Stützflächen des Lagerkörpers drücken. Der Anschlagkontakt kann direkt zwischen einer der Stützflächen des Rotationsglieds und einer der Stützflächen des Lagerkörpers gebildet sein oder indirekt über einen oder mehrere Zwischenkörper. Ein solcher Zwischenkörper kann insbesondere von einem Wälzlager gebildet werden. In einer besonders bevorzugten einfachen Ausführung sind die beiden betreffenden Stützflächen der Drehlagerung jedoch unmittelbar miteinander in Anschlagkontakt, d.h. sie bilden miteinander ein Gleitdrehlager.

Die Spielreduziereinrichtung bildet in einer bevorzugten weiteren Ausführung oder auch in einer Weiterbildung der Ausführung mit Feder wenigstens eine der genannten Stützflächen, vorzugsweise genau eine der Stützflächen. Falls die Spielreduziereinrichtung eine der axial steif mit dem Lagerkörper verbundenen Stützflächen bildet, wie dies bevorzugt wird, ist die Spielreduziereinrichtung mit dem Lagerkörper in einem Einstelleingriff. In diesem Fall ist die Spielreduziereinrichtung entlang eines durch den Einstelleingriff vorgegebenen Verstellwegs relativ zu dem Lagerkörper verstellbar und wird in solch eine Einstellposition verstellt sowie in der Einstellposition relativ zu dem Lagerkörper axial so gesichert, dass ein Axialspiel der Drehlagerung reduziert, vorzugsweise eliminiert wird. In dem Einstelleingriff mit dem Lagerkörper werden somit die erste Stützfläche und die zweite Stützfläche relativ zueinander axial verstellt und in der Einstellposition relativ zueinander axial gesichert. Falls die Spielreduziereinrichtung eine der axial steif mit dem Rotationsglied verbundenen Stützflächen bildet, ist sie mit dem Rotationsglied in einem entsprechenden Einstelleingriff, in dem die dritte Stützfläche und die vierte Stützfläche relativ zueinander in solch eine Einstellposition verstellt und in der Einstellposition relativ zueinander axial so gesichert sind, dass das Axialspiel der Drehlagerung reduziert und vorzugsweise eliminiert wird. Die in dem Einstelleingriff verfügbare Verstellweglänge sollte so groß sein, dass die Einstellposition nicht durch Anschlagen der Spielreduziereinrichtung an demjenigen Körper bestimmt wird, mit dem sie in dem Einstelleingriff ist.

Die Drehlagerung kann ein Wälzlager umfassen. Herkömmliche Wälzlager weisen ein inhärentes Axialspiel auf. Auch das Wälzlager der erfindungsgemäßen Vorrichtung wird letztlich auf eine bestimmte Passung und somit toleranzbehaftet gefertigt. Nach der Erfindung kann in bevorzugten Ausführungen mit Wälzlager jedoch eine der Stützflächen relativ zu den anderen axial verstellt und dadurch das aufgrund der unvermeidbaren Toleranzen inhärente Axialspiel reduziert werden. Eine der axial steif mit dem Lagerkörper verbundenen Stützflächen stützt das Wälzlager in einer Axialrichtung ab. Eine dieser Stützfläche axial zugewandte Stützfläche, die axial steif mit dem Rotationsglied verbunden ist, stützt das Wälzlager in die andere Axialrichtung ab. Das Wälzlager weist einen in Bezug auf die Rotationsachse des Rotationsglieds radial äußeren Lagerring und einen radial inneren Lagerring auf. Vorteilhafterweise ist das Wälzlager nur mit einem der Ringe axial an dem Rotationsglied und nur mit dem anderen der Ringe axial an dem Lagerkörper abgestützt. Die Drehlagerung kann ein weiteres Wälzlager umfassen, das in gleicher Weise zwischen den beiden verbleibenden Stützflächen der Drehlagerung angeordnet und von diesen Stützflächen axial abgestützt wird. Auch das weitere Wälzlager weist einen in Bezug auf die Rotationsachse radial inneren und einen radial äußeren Lagerring auf. Vorzugsweise ist auch bei dem weiteren Wälzlager nur der eine der Lagerringe axial an dem Rotationsglied und nur der andere der Lagerringe axial an dem Lagerkörper abgestützt. Die mit dem Lagerkörper axial steif verbundenen Stützflächen sollten in einer ersten axialen Flucht und/oder die mit dem Rotationsglied axial steif verbundenen Stützflächen sollten in einer zweiten axialen Flucht liegen. Falls die Drehlagerung zwei Wälzlager aufweist, sind in solchen Ausführungen entweder die inneren Lagerringe oder vorzugsweise die äußeren Lagerringe beider Wälzlager axial an den dem Lagerkörper zugeordneten Stützflächen und dementsprechend entweder die äußeren Lagerringe oder die inneren Lagerringe beider Wälzlager axial an den dem Rotationsglied zugeordneten Stützflächen abgestützt.

Die Drehlagerung kann auch eine reine Gleitlagerung sein. Die jeweils paarweise einander zugewandten Stützflächen des Rotationsglieds und des Lagerkörpers gleiten in solch einer Ausbildung unmittelbar aufeinander ab, wenn das Rotationsglied seine Rotationsbewegung relativ zu dem Lagerkörper ausführt. Schließlich kann die Drehlagerung auch eine Kombination einer Gleitlagerung und einer Wälzlagerung sein.

Der Einstelleingriff der Spielreduziereinrichtung mit entweder dem Rotationsglied oder vorzugsweise dem Lagerkörper ist in bevorzugter Ausführung form- und kraftschlüssig. Der Einstelleingriff ist vorzugsweise kontinuierlich in dem Sinne, dass das Axialspiel kontinuierlich im Einstelleingriff verringert werden kann. Der Einstelleingriff ist über die Verstellweglänge vorzugsweise selbsthemmend. Ein bevorzugtes Beispiel eines kontinuierlichen Einstelleingriffs ist ein Gewindeeingriff zwischen der Spielreduziereinrichtung und vorzugsweise dem Lagerkörper oder zwischen der Spielreduziereinrichtung und dem Rotationsglied.
Der Gewindeeingriff bietet ferner den Vorteil, dass die Spielreduziereinrichtung durch den Einstelleingriff selbst in jeder Position, die sie über den gesamten, im Einstelleingriff verfügbaren Verstellweg einnimmt, axial gestützt wird, und daher mit dem Körper axial steif verbunden ist, mit dem sie in dem Einstelleingriff ist.

Eine Sicherung der Spielreduziereinrichtung kann im Falle des Gewindeeingriffs in der Einstellposition allein durch Selbsthemmung bewirkt werden. Vorzugsweise ist die Spielreduziereinrichtung in ihrer Einstellposition jedoch stoffschlüssig fixiert, auch wenn der Einstelleingriff wie bevorzugt ein Gewindeeingriff ist.

Die verstellbare Spielreduziereinrichtung ist vorzugsweise eingliedrig als ein einziges Einstellglied gebildet, das in dem Einstelleingriff entsprechend dem zu reduzierenden Axialspiel zumindest axial steif ist. Im Falle einer mehrgliedrigen Spielreduziereinrichtung, sollte solch eine Spielreduziereinrichtung zumindest dann axial in sich steif sein, wenn sie in der Einstellposition gesichert ist. So könnte beispielsweise eine zweigliedrige Spielreduziereinrichtung ein erstes Einstellglied aufweisen, das in einem Einstelleingriff mit dem Lagerkörper ist, und ein zweites Einstellglied, das in einem Einstelleingriff mit dem Rotationsglied ist. Die beiden Einstellglieder würden bis in die Einstellposition hinein axial relativ zueinander verstellt und müssten in der Einstellposition axial aneinander festgelegt werden oder sich selbsttätig aneinander festlegen, um die axiale Steifigkeit zu erhalten.

Vorzugsweise treibt das Rotationsglied das Translationsglied an. Das Translationsglied kann bei der Translationsbewegung unmittelbar auf das in dem Reservoir befindliche Produkt wirken, indem es beispielsweise selbst einen Hubkolben bildet oder mit einem Hubkolben permanent verbunden ist. Es kann aber auch nur lose gegen einen Hubkolben drücken. Möglich ist auch eine Ausführung, in der das Translationsglied erst über ein Übertragungsglied oder mehrere Übertragungsglieder auf ein unmittelbar auf das Produkt wirkendes Förderelement, beispielsweise einen Hubkolben, wirkt, wenn es die Translationsbewegung ausführt. Das Rotationsglied kann insbesondere die Eingangsstufe einer teleskopierbaren Fördereinrichtung bilden, wie sie in der diesbezüglich hiermit in Bezug genommenen DE 197 17 107 A beschrieben wird. Grundsätzlich könnte aber auch das Translationsglied das Rotationsglied antreiben, insbesondere wenn das Rotationsglied erst über ein oder mehrere Übertragungsglieder auf das Produkt wirkt.

Die Kopplung zwischen dem Rotationsglied und dem Translationsglied ist vorzugsweise ein Flankeneingriff, zu dessen Bildung das Rotationsglied und das Translationsglied je wenigstens eine Eingriffsflanke aufweisen. Vorzugsweise sind sowohl die wenigstens eine Eingriffsflanke des Rotationsglieds unmittelbar an dem Rotationsglied als auch die wenigstens eine Eingriffsflanke des Translationsglieds unmittelbar an dem Translationsglied geformt.

Flankeneingriffe, wie sie insbesondere von Gewindeeingriffen, aber auch anderen Kurvenführungen bekannt sind, weisen fertigungsbedingt quer zu den Eingriffsflanken ein axiales Spiel auf, das der Dosiergenauigkeit abträglich sein kann. Ein axiales Spiel kann sich insbesondere bei Auftreten des sogenannten Syphoning-Effekts bemerkbar machen, d.h. wenn es im Behältnis aufgrund der Ausströmverhältnisse zu einer Saugsituation kommt.

Es kann deshalb in bevorzugten Ausführungen eine weitere Spielreduziereinrichtung vorgesehen sein, die sowohl mit dem Rotationsglied als auch mit dem Translationsglied je in einem Einstelleingriff ist, in dem die weitere Spielreduziereinrichtung relativ zu dem Rotationsglied und dem Translationsglied in solch eine Einstellposition verstellt und in der Einstellposition so gesichert ist, dass das dem Flankeneingriff inhärente Axialspiel gegenüber den bekannten Kopplungen reduziert oder vorzugsweise gänzlich eliminiert ist. Der Einstelleingriff mit einem der beiden Glieder aus Rotationsglied und Translationsglied entspricht dem Flankeneingriff zwischen dem Rotationsglied und dem Translationsglied. Der andere Einstelleingriff gibt die Verstellbewegung der Spielreduziereinrichtung entlang ihres Verstellwegs vor.

Der Einstelleingriff mit dem Rotationsglied ist vorzugsweise form- und kraftschlüssig, besonders bevorzugt ist der Einstelleingriff ein Gewindeeingriff. Bezüglich des Einstelleingriffs mit dem Translationsglied gilt vorzugsweise das Gleiche. Die Ausbildung beider Einstelleingriffe der weiteren Spielreduziereinrichtung je als Gewindeeingriff bietet sich insbesondere dann an, wenn der Flankeneingriff zwischen dem Rotationsglied und dem Translationsglied ebenfalls eine Gewindeeingriff ist, wie die Erfindung es bevorzugt. Es ist jedoch beispielsweise auch möglich, den Einstelleingriff, der die Verstellbewegung vorgibt, als Eingriff eines Eingriffsglieds der Spielreduziereinrichtung in eine Führungsbahn rein formschlüssig zu gestalten und mittels einer Elastizitätskraft den anderen der beiden Einstelleingriffe form- und kraftschlüssig zu bilden. Der die Verstellbewegung vorgebende Einstelleingriff der weiteren Spielreduziereinrichtung ist in bevorzugten Ausführungen kontinuierlich in dem Sinne, dass das Axialspiel zwischen dem Rotationsglied und dem Translationsglied von seiner fertigungsbedingten Ausgangsgröße bis vorzugsweise auf den Wert Null kontinuierlich in diesem Einstelleingriff verringert werden kann, wie dies beispielsweise der bevorzugte Gewindeeingriff ermöglicht. Falls die Fördereinrichtung teleskopierbar ist, wird je eine weitere Spielreduziereinrichtung vorteilhafterweise zwischen jedem Paar von in Flankeneingriffen befindlichen Teleskopstufen vorgesehen.

In bevorzugten Ausführungen ist die weitere Spielreduziereinrichtung an einem der Glieder aus Rotationsglied und Translationsglied gegen Axialbewegungen relativ zu dem betreffenden Glied gesichert. Die Sicherung kann insbesondere so gestaltet sein, dass die weitere Spielreduziereinrichtung die Rotationsbewegung mitmacht, wenn die Sicherung zwischen dem Rotationsglied und der Spielreduziereinrichtung besteht, und die Translationsbewegung mitmacht, wenn die Sicherung zwischen der weiteren Spielreduziereinrichtung und dem Translationsglied besteht.

Im beispielhaften Fall des Gewindeeingriffs kann die Sicherung der weiteren Spielreduziereinrichtung in einem der Einstelleingriffe in der Einstellposition allein durch Selbsthemmung bewirkt werden. Vorzugsweise ist die weitere Spielreduziereinrichtung in ihrer Einstellposition jedoch stoffschlüssig gesichert, auch wenn der gesicherte Einstelleingriff ein Gewindeeingriff ist. Die stoffschlüssige Sicherung erfolgt vorzugsweise in dem Einstelleingriff mit dem Rotationsglied. Eine Sicherung an dem Translationsglied wäre grundsätzlich jedoch in kinematischer Umkehr ebenfalls möglich. Anstatt die Sicherung allein in einem einzigen der beiden Einstelleingriffe zu realisieren, kann die Sicherung auch erst im Zusammenwirken der weiteren Spielreduziereinrichtung mit dem Antriebsglied und dem Abtriebsglied realisiert werden, in diesem Fall vorzugsweise dadurch, dass die weitere Spielreduziereinrichtung sowohl an dem Antriebsglied als auch an dem Abtriebsglied elastisch abgestützt ist.

Bevorzugte Ausgestaltungen der Erfindung werden auch in den Unteransprüchen und durch die Kombinationen der Unteransprüche beschrieben.

Ausführungsbeispiele der Erfindung werden nachfolgend anhand von Figuren erläutert. An den Ausführungsbeispielen offenbar werdende Merkmale bilden je einzeln und in jeder Merkmalskombination die Gegenstände der Ansprüche und die vorstehend beschriebenen Ausgestaltungen vorteilhaft weiter. Es zeigen:
- Figur 1: eine Verabreichungsvorrichtung eines ersten Ausführungsbeispiels in einem Längsschnitt,
- Figur 2: ein Teil der Verabreichungsvorrichtung in einem anderen Längsschnitt,
- Figur 3: ein vergrößertes Detail der Figur 2,
- Figur 4: eine Spielreduziereinrichtung in einer alternativen Ausführung,
- Figur 5: ein Einstellglied der Spielreduziereinrichtung der Figur 4,
- Figur 6: eine Verabreichungsvorrichtung eines zweiten Ausführungsbeispiels in einem Längsschnitt,
- Figur 7: ein Detail X der Figur 6,
- Figur 8: einen Federring einer Spielreduziereinrichtung des zweiten Ausführungsbeispiels,
- Figur 9: Komponenten der Verabreichungsvorrichtung des zweiten Ausführungsbeispiels in einer Explosionsdarstellung und
- Figur 10: das Translationsglied des zweiten Ausführungsbeispiels.

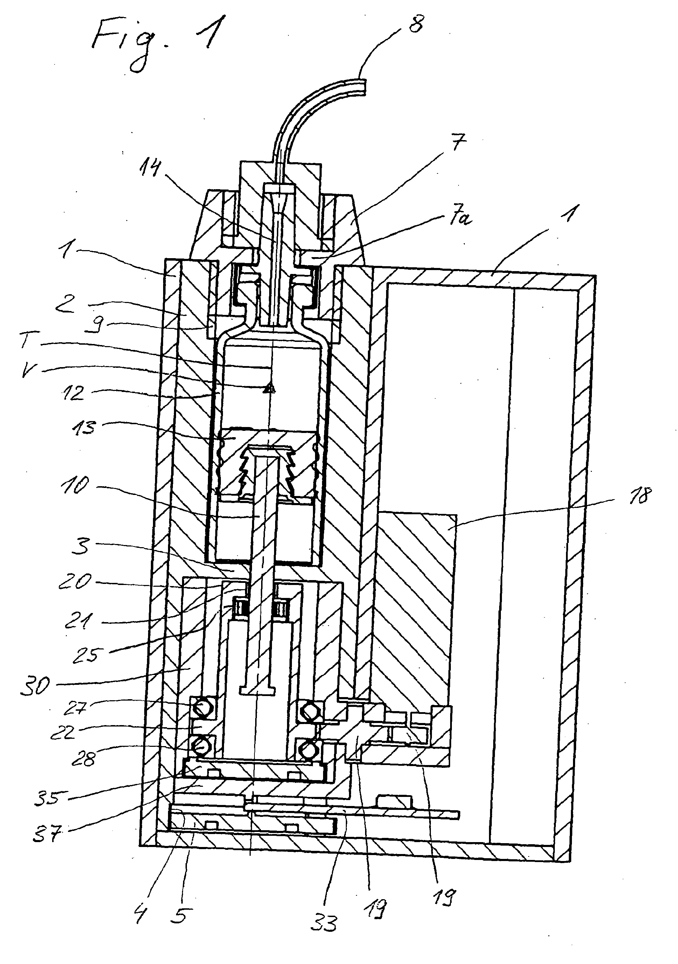

Figur 1 zeigt in einem Längsschnitt als Beispiel einer Verabreichungsvorrichtung ein Infusionsgerät. Das Gerät weist ein Gehäuse mit einer ersten Gehäusestruktur 1, und einer zweiten Gehäusestruktur 2, ein mit einem injizierbaren Produkt gefiilltes Behältnis 12 und eine Fördereinrichtung auf, die das Produkt dosiert aus dem Behältnis 12 und durch einen sich anschließenden Katheter 8 fördert, um es zu verabreichen. Die Verabreichung kann insbesondere subkutan vorgenommen werden, wie dies beispielsweise in der Diabetestherapie üblich ist. Die erste Gehäusestruktur 1 umgibt die zweite Gehäusestruktur 2 und wird im folgenden als Hüllstruktur 1 bezeichnet. Die zweite Gehäusestruktur 2 stützt Komponenten des Infusionsgeräts und wird im folgenden als Stützstruktur 2 bezeichnet.

Das Behältnis 12 weist an einem vorderen Ende einen Auslass 14 auf, über den der Behältnisinnenraum mit dem Katheter 8 verbunden ist. In dem Behältnis 12 ist ein Kolben 13 entlang einer Translationsachse T in eine Förderrichtung V auf den Behältnisauslass 14 zu bewegbar aufgenommen. Das Behältnis 12 ist an seinem rückwärtigen Ende offen. Allerdings dichtet der Kolben 13 das Behältnis 12 zu dem rückwärtigen Ende hin ab.

Die Hüllstruktur 1 bildet eine feste Hülle, in der die Stützstruktur 2, die von einem Stützkörper 2 einstückig gebildet wird, eingesetzt und befestigt ist und ein Chassis des Gehäuses bildet. Die Hüllstruktur 1 und der Stützkörper 2 bilden das Gehäuse der Vorrichtung zumindest im Wesentlichen. Der Stützkörper 2 bildet einen ersten Aufnahmeraum, in dem das Behältnis 12 eingesetzt ist, und einen zweiten Aufnahmeraum für die Fördereinrichtung. Das Behältnis 12 ist mit seinem hinteren freien Rand auf Anschlag gegen einen, eine Stützschulter bildenden, nach radial einwärts ragenden Stützsteg 3 des Stützkörpers 2. Der von dem Stützkörper 2 gebildete erste Aufnahmeraum weist an dem vorderen Ende eine Öffnung auf, durch die das Behältnis 12 eingesetzt wird. Nach dem Einsetzen des Behältnisses 12 wird die Öffnung mit einem Deckel 7 verschlossen. Der Deckel 7 ist mit dem Stützkörper 2 verschraubt, der hierfür mit einem Gewinde 9 versehen ist. Der Stützkörper 2 und der Deckel 7 könnten auch mit je einer anderen Eingriffseinrichtung für den lösbaren Eingriff versehen sein, beispielsweise mit kooperierenden Rasteinrichtungen. Der Deckel 7 bildet ferner das Verbindungselement zwischen dem Katheter 8 und dem Auslass 14 des Behältnisses 12. Der Deckel 7 weist zu der Stützschulter des Stützstegs 3 eine Gegenstützschulter an einem Gegenstützsteg 7a auf, die gegen einen vorderen Rand des Behältnisses 12 und dadurch das Behältnis 12 auf Anschlag gegen den Stützsteg 3 drückt, so dass das Behältnis 12 relativ zu dem Stützkörper 2 axial festgelegt ist. Der Deckel 7 bildet somit ein vorderes Abschlusselement 7a, und der Stützsteg 3 bildet ein hinteres Abschlusselement 3 des ersten Aufnahmeraums. Der erste Aufnahmeraum ist ferner so geformt, dass das Behältnis 12 die korrekte Position und Ausrichtung in Bezug auf die Translationsachse T hat. Im Ergebnis stützt der Stützkörper 2 das Behältnis 12 axial in und gegen die Förderrichtung V formschlüssig, d.h. durch Kontakt mit den als Anschlägen dienenden Stegen 3 und 7a, ab. Die Fördereinrichtung wird in gleicher Weise axial zwischen anderen Anschlägen des Stützkörpers 2 abgestützt.

Die Fördereinrichtung umfasst den Kolben 13, ein Abtriebsglied 10, ein Antriebsglied 20 und einen motorischen Drehantrieb. Das Abtriebsglied 10 bildet ein Translationsglied der Fördereinrichtung, und das Antriebsglied 20 bildet ein Rotationsglied der Fördereinrichtung.

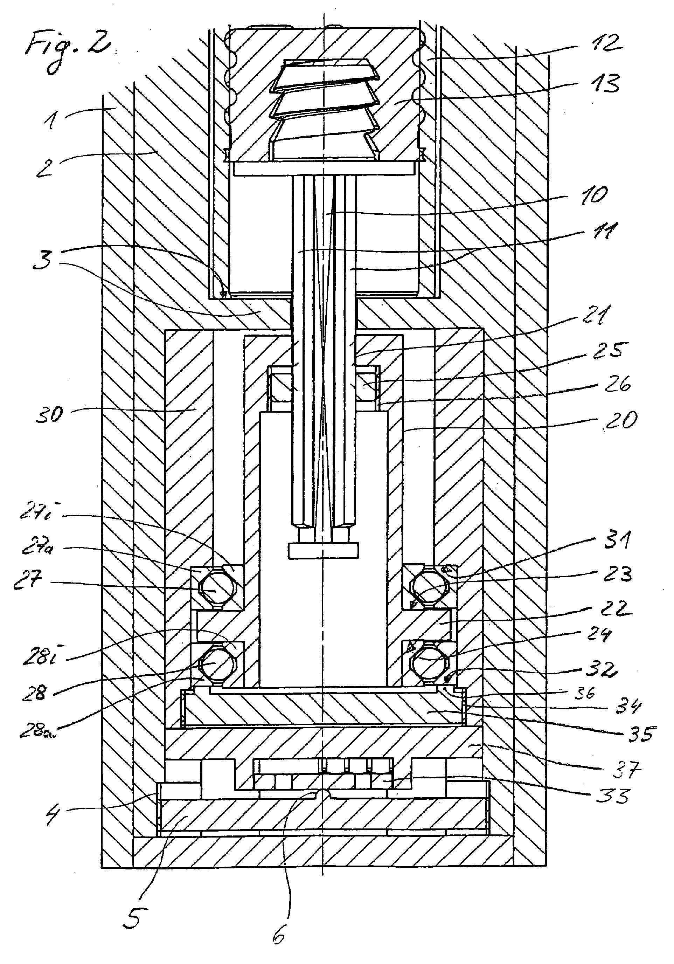

Das Abtriebsglied 10 ist eine Kolbengewindestange, d. h. eine Kolbenstange, die mit einem Gewinde versehen ist. Im Ausführungsbeispiel ist das Abtriebsglied 10 mit einem Außengewinde 11 versehen, das in dem Längsschnitt der Figur 2 erkennbar ist. Das Abtriebsglied 10 durchragt den Stützsteg 3, so dass es in den ersten Aufnahmeraum und den zweiten Aufnahmeraum des Stützkörpers 2 hinein ragt. An seinem vorderen Ende ist das Abtriebsglied 10 mit dem Kolben 13 verschraubt. Die Schraubverbindung wird mit dem Einsetzen des Behältnisses hergestellt. Der Stützsteg 3 führt das Abtriebsglied 10 linear entlang der Translationsachse T. Der Stützsteg 3 sichert das Abtriebsglied 10 so gegen eine Verdrehung relativ zu dem Stützkörper 2. Das Gewinde 11 wird im Ausführungsbeispiel hierfür von wenigstens einer axialen Nut oder Abplattung durchbrochen, in die der Stützsteg 3 eingreift.

Das Antriebsglied 20 ist gänzlich in dem zweiten Aufnahmeraum des Stützkörpers 2 angeordnet. Es ist rotationssymmetrisch zur Translationsachse T. Es ist hülsenformig und kann daher auch als Antriebshülse bezeichnet werden. Das Antriebsglied 20 bildet an einem vorderen Hülsenende einen nach radial einwärts ragenden Steg, den das Abtriebsglied 10 durchragt. Das Antriebsglied 20 bildet an dem einwärts ragenden Steg ein Innengewinde 21, das mit dem Außengewinde 11 des Abtriebsglieds 10 in einem Gewindeeingriff ist.

Das Antriebsglied 20 ist um die Translationsachse T drehbar und relativ zu dem Stützkörper 2 axial nicht bewegbar gelagert. Es weist in einem hinteren Bereich einen nach radial auswärts ragenden, umlaufenden Steg 22 auf, der mit einer Außenverzahnung versehen ist. Über die Außenverzahnung wird das Antriebsglied 20 um die Translationsachse T drehangetrieben. Seinen Drehantrieb erhält es von einem Drehmotor 18, der in einem weiteren Aufnahmeraum aufgenommen ist. Der weitere Aufnahmeraum wird von der Hüllstruktur 1 gebildet und ist von den beiden Aufnahmeräumen des Stützkörpers 2 getrennt. Der Motor 18 treibt das Antriebsglied 20 über ein Zahnradgetriebe mit Stirnzahnrädern 19 an, von denen ein Ausgangszahnrad 19 mit der Außenverzahnung des Antriebsglieds 20 kämmt. Infolge des Gewindeeingriffs zwischen dem Antriebsglied 20 und dem Abtriebsglied 10 und der Linearführung des Abtriebsglieds 10, wird bei einem Drehantrieb des Antriebsglieds 20 durch dessen rotatorische Antriebsbewegung eine axiale Abtriebsbewegung des Abtriebsglieds 10 in die Förderrichtung V bewirkt. Das durch die Kolbenbewegung verdrängte Produkt wird über den Katheter 8 ausgeschüttet und auf diese Weise verabreicht.

Wie jeder Gewindeeingriff ist auch der Gewindeeingriff als solcher zwischen dem Abtriebsglied 10 und dem Antriebsglied 20 mit einem Axialspiel behaftet. Die Dosiergenauigkeit der Ausschüttung ist daher zumindest im Ausmaß dieses inhärenten Axialspiels mit einer Ungenauigkeit behaftet. Im Falle beispielsweise eines Ansaugens des Kolbens 13 aufgrund Syphoning oder bei mechanischen Stößen oder Druckdifferenzen zwischen dem Gehäuseinnenraum und der Umgebung kann es daher vorkommen, dass die Flanken des Außengewindes 11 des Abtriebsglieds 10 von den vortreibenden Gewindeflanken des Gewindes 21 abheben. Die axiale Position des Kolbens 13 ist daher im Ausmaß des Axialspiels der Gewinde 11 und 21 unbestimmt.

Allerdings ist eine Spielreduziereinrichtung vorgesehen, die mittels eines Einstellglieds 25 gebildet ist und die das Axialspiel zwischen dem Abtriebsglied 10 und dem Antriebsglied 20 nahezu eliminiert.

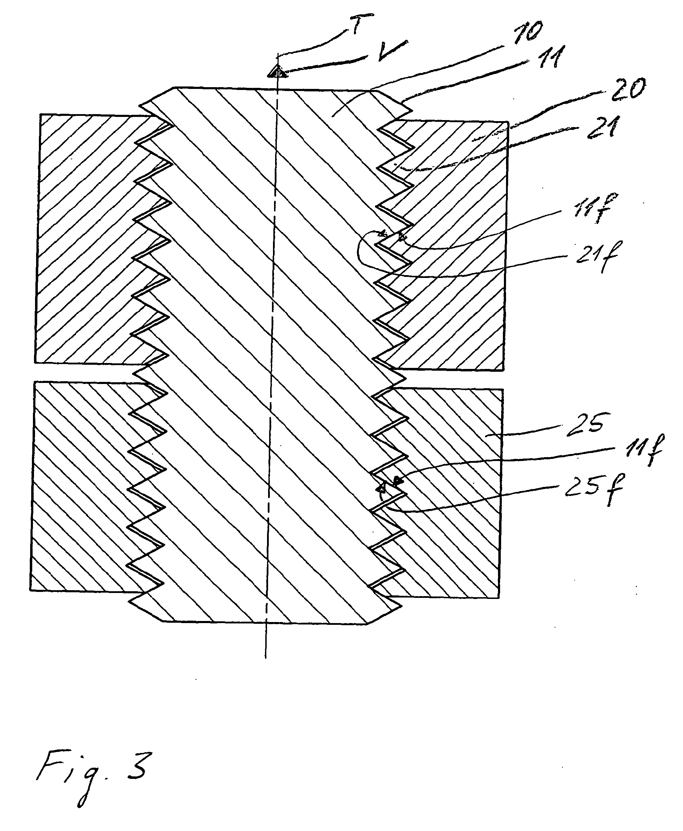

Aus den Figuren 2 und 3 sind Konstruktion und Wirkung der Spielreduziereinrichtung am besten ersichtlich. Die Spielreduziereinrichtung wird von einem einstückigen Einstellglied 25 gebildet. Das Einstellglied 25 ist in einem Einstelleingriff mit dem Abtriebsglied 10 und in einem weiteren Einstelleingriff mit dem Antriebsglied 20. Das Einstellglied 25 und die beiden Einstelleingriffe sind so gestaltet, dass das Axialspiel zwischen den Gewinden 11 und 21 deutlich reduziert oder vorzugsweise gänzlich aufgehoben wird.

Im Ausführungsbeispiel ist das Einstellglied 25 als Gewindemutter mit einem Innengewinde und einem Außengewinde gebildet. Mit seinem Innengewinde ist das Einstellglied 25 in einem Gewindeeingriff mit dem Außengewinde 11 des Abtriebsglieds 10. Mit seinem Außengewinde ist es in einem Gewindeeingriff mit einem Innengewinde 26 des Antriebsglieds 20. Das Innengewinde 26 ist dem Außengewinde 11 zugewandt und axial unmittelbar hinter dem Gewinde 21 gebildet. Das Innengewinde und das Außengewinde des Einstellglieds 25 liegen auf der gleichen axialen Höhe, so dass das Einstellglied 25 axial dünn sein kann und die Spielreduziereinrichtung dementsprechend axial, d. h. entlang der Translationsachse T, kurz baut. Das Innengewinde 26 muss lediglich so lang sein, dass ein sicherer Einstelleingriff mit dem Einstellglied 25 gewährleistet ist und das Einstellglied 25 darüber hinaus in diesem Einstelleingriff noch so weit verstellt werden kann, dass die angestrebte Reduzierung des Axialspiels der Gewinde 11 und 21 herbeigeführt werden kann. Das Innengewinde 26 weist eine Steigung auf, die es erlaubt, das Einstellglied 25 bei bestehendem Gewindeeingriff zwischen den Gewinden 11 und 21 und zwischen dem Gewinde 11 und dem Innengewinde des Einstellglieds 25 im Gewindeeingriff mit dem Innengewinde 26 zu verstellen.

Figur 3 zeigt in vergrößerter Darstellung den Gewindeeingriff der Gewinde 11 und 21 und den Einstelleingriff zwischen dem Gewinde 11 des Abtriebsglieds 10 und dem Innengewinde des Einstellglieds 25. Das Innengewinde des Einstellglieds 25 weist die gleiche Steigung wie das Außengewinde 11 auf. Vorzugsweise ist die Steigung des Außengewindes des Einstellglieds 25 und des Innengewindes 26 größer oder kleiner als die Steigung der Gewinde 11 und 21, allerdings so geringfügig, dass die Verstellung des Einstellglieds 25 im Einstelleingriff möglich ist.

Für die Axialspielreduzierung ist das Einstellglied 25 in seinem Einstelleingriff mit dem Abtriebsglied 10 so eingestellt, dass seine bezüglich der Förderrichtung V rückwärtigen Gewindeflanken 25f in Kontakt mit den vorderen Gewindeflanken 11f des Außengewindes 11 sind, während gleichzeitig die vorderen Flanken 21f des antreibenden Gewindes 21 in Kontakt mit den rückwärtigen Flanken 11f des Gewindes 11 des Abtriebsglieds 10 sind. Hierfür wird das Einstellglied 25 in seinem Einstelleingriff mit dem Antriebsglied 20 relativ zu dem Antriebsglied 20 gegen die Förderrichtung V verstellt, bis dieser Zustand des Flankenkontakts hergestellt ist. In diesem Zustand wird das Einstellglied 25 an dem Antriebsglied 20 fixiert und dadurch gesichert. Die Sicherung wird im Ausführungsbeispiel adhäsiv hergestellt, indem ein Adhäsionsmittel in den Einstelleingriff des Einstellglieds 25 mit dem Antriebsglied 20 eingebracht ist. Andere Möglichkeiten der stoffschlüssigen Verbindung zwischen dem Einstellglied 25 und dem Antriebsglied 20 sind jedoch ebenfalls denkbar, beispielsweise eine Laserverschweißung in der Einstellposition. Falls das Innengewinde 26, wie bevorzugt, eine andere Steigung als die Gewinde 11 und 21 hat, kann bereits hierdurch allein oder in Kombination mit einer stoffschlüssigen Verbindung die axiale Sicherung erzielt werden. Die Einstellposition sollte so gewählt werden, dass durch die Spielreduzierung keine oder jedenfalls keine praktisch relevanten Presskräfte auf das Abtriebsglied 10 ausgeübt werden. Die Einstellposition ist daher so gewählt, dass in dem Gewindeeingriff der Gewinde 11 und 21 ein sehr geringes Restspiel verbleibt, das jedoch deutlich kleiner als das diesem Eingriff allein, d. h. ohne Spielreduzierung, inhärente Gewindespiel ist.

Im Ausführungsbeispiel ist die Einstellposition des Einstellglieds 25 so gewählt, dass das Gewinde 21 das Antriebsgewinde des Antriebsglieds 20 bleibt. Die Einstellposition könnte jedoch auch so gewählt sein, dass bei der Einstellung das Einstellglied 25 gegen die rückwärtigen Gewindeflanken des Gewindes 11 bewegt wird und in diesem Fall das Einstellglied 25 den Vortrieb des Abtriebsglieds 10 übernimmt. Bevorzugt wird jedoch die für das Ausführungsbeispiel gewählte und aus Figur 3 ersichtliche Einstellposition des Einstellglieds 25.

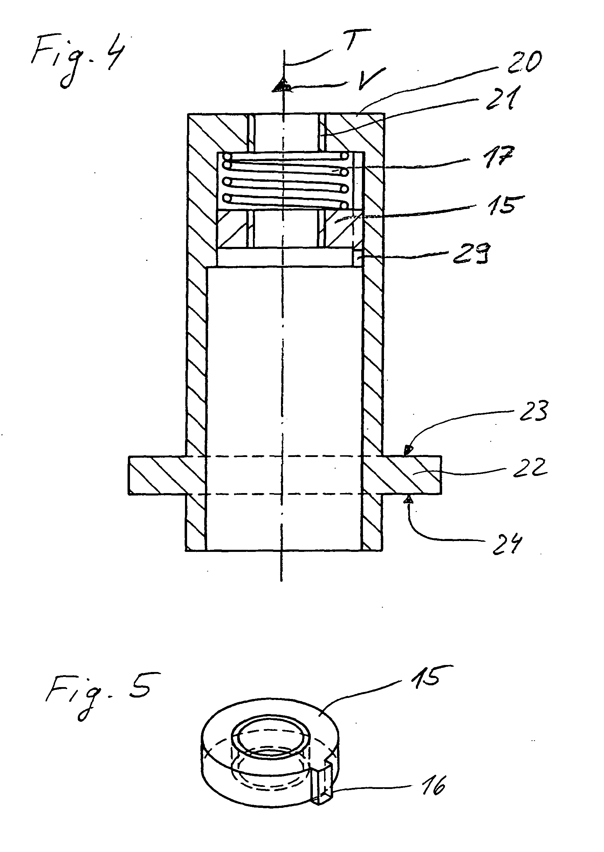

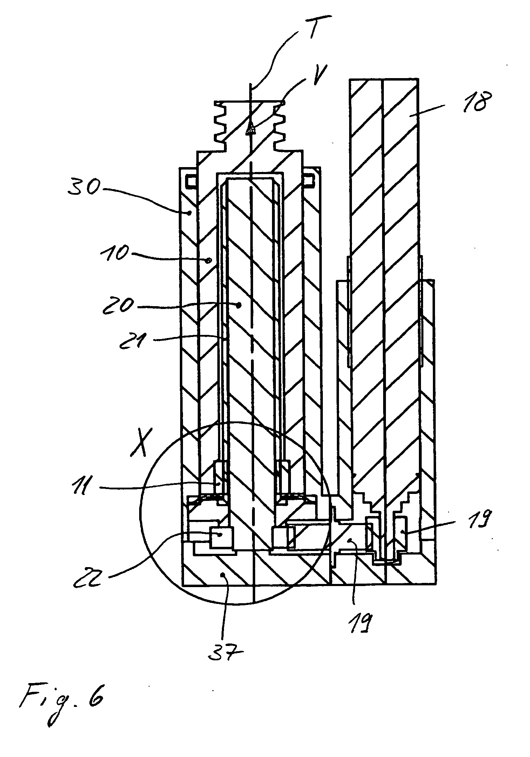

Ein alternatives Ausführungsbeispiel einer Spielreduziereinrichtung ist aus den Figuren 4 und 5 erkennbar. Im Vergleich zu den Komponenten des Ausführungsbeispiels der Figuren 1 bis 3 sind lediglich das Antriebsglied 20 und das im alternativen Ausführungsbeispiel mit "15" gekennzeichnete Einstellglied modifiziert, während die übrigen Komponenten, insbesondere das Abtriebsglied 10, unverändert sind. Als weiteren Unterschied umfasst die alternative Spielreduziereinrichtung zusätzlich ein elastisches Rückstellelement 17 in Form einer mechanischen Druckfeder.

Das Einstellglied 15 ist wie bereits das Einstellglied 25 in die das Antriebsglied 20 bildende Hülse eingesetzt. Allerdings ist das Einstellglied 15 mit dem Antriebsglied 20 axial linear verschiebbar und verdrehgesichert verbunden. Der Einstelleingriff des Einstellglieds 15 mit dem Antriebsglied 20 ist daher eine Linearführung. Die Linearführung wird von einer axialen, geraden Führungsbahn 29 an der Mantelinnenfläche des Abtriebsglieds und einem in die Führungsbahn 29 eingreifenden Eingriffsglied 16 (Fig. 5) des Einstellglieds 15 gebildet. Die Führungsbahn 29 wird in die Förderrichtung V von dem nach radial einwärts ragenden Steg des Antriebsglieds 20 begrenzt, der das Antriebsgewinde 21 bildet. Rückwärtig ist die Führungsbahn 29 offen, so dass das Einstellglied 15 eingeschoben werden kann. Zuvor wird noch das Rückstellelement 17 eingesetzt. Das Rückstellelement 17 ist in Förderrichtung an dem das Antriebsgewinde 21 bildenden Steg des Antriebsglieds 20 und gegen die Förderrichtung an dem Einstellglied 15 abgestützt. Figur 4 zeigt diesen Zustand vor dem Zusammenbau mit dem Abtriebsglied 10.

Für den Zusammenbau wird zuerst das Einstellglied 15 mit dem Rückstellelement 17 in das Antriebsglied 20 in den Einstellgriff mit der Führungsbahn 29 eingesetzt und mit einer gewissen Kraft gegen das Rückstellelement 17 gedrückt. Das Abtriebsglied 10 wird anschließend zuerst mit dem Einstellglied 15 und dann mit dem Antriebsgewinde 21 verschraubt. Im Einstelleingriff drücken die rückwärtigen Gewindeflanken des Innengewindes des Einstellglieds 15 mit einer Elastizitätskraft gegen die in die Förderrichtung V weisenden Gewindeflanken des Gewindes 11 des Abtriebsglieds 10. Im Ergebnis wird für den Gewindeeingriff der Gewinde 11 und 21 durch die zwei Einstelleingriffe des Einstellglieds 15 der gleiche Zustand erhalten, wie er in Figur 3 dargestellt ist. In der alternativen Spielreduziereinrichtung wird das Einstellglied 15 somit durch die Elastizitätskraft des Rückstellelements 17 in der Einstellposition gesichert.

Das Infusionsgerät des Ausführungsbeispiels weist als eine Besonderheit, wie sie jedoch grundsätzlich aus der DE 198 40 992 A bekannt ist, eine Okklusionserkennung auf, der ebenfalls ein Axialspiel inhärent ist, unter dem die Dosiergenauigkeit leiden kann. Dieses inhärente, zweite Axialspiel hat seine Ursache darin, dass die gesamte Fördereinrichtung, insbesondere das Abtriebsglied 10 und das Antriebsglied 20 über einen Sensor 33 an dem Stützkörper 2 axial abgestützt ist. Der Sensor 33 dient der Ermittlung der Kraft, die erforderlich ist, um den Kolben 13 entlang der Translationsachse T zu bewegen. Der Sensor kann beispielsweise auf einer Dehnungsmessung beruhen. Letztlich wird mittels des Sensors 33 eine den Fluiddruck in dem Behältnis 12 repräsentierende, physikalische Größe gemessen, um bei der Produktverabreichung eine etwaige Okklusion oder ein etwaiges Leck möglichst frühzeitig zu erkennen. Was die Okklusions- und/oder Leckerkennung und den Sensor 33 anbetrifft, wird im Folgenden lediglich auf die das Axialspiel betreffenden Aspekte eingegangen und im Übrigen auf die als Beispiel in Bezug genommene DE 198 40 992 A verwiesen.

Für die Okklusions- und/oder Leckerkennung ist die Fördereinrichtung, wie bereits erwähnt, über den Sensor 33 axial abgestützt. Dies bedeutet, dass das Antriebsglied 20, an dem sich das Abtriebsglied 10 im Antriebseingriff der Gewinde 11 und 21 axial abstützt, nicht axial steif mit dem Stützkörper 2 verbunden, sondern relativ zu dem Stützkörper 2 axial bewegbar gelagert ist, um nämlich den Fluiddruck in dem Behältnis 12, genauer gesagt den Differenzdruck zur Umgebung, ermitteln zu können. Um die axial bewegbare Lagerung zu erhalten, ist das Antriebsglied 20 in einem Lagerkörper 30 drehgelagert und an dem Lagerkörper 30 axial gesichert. Der Lagerkörper 30 ist in den zweiten Aufnahmeraum des Stützkörpers 2 eingesetzt und gegen Verdrehung gesichert. Der Stützkörper 2 führt den Lagerkörper 30 axial durch Gleitkontakt. Der Lagerkörper 30, und damit zusammen das Abtriebsglied 10 und das Antriebsglied 20, ist über den Sensor 33 an dem Stützkörper 2 axial so abgestützt, dass der Sensor 33 die gesamte, zwischen dem Lagerkörper 30 und dem Stützkörper 2 wirkende und gegen die Förderrichtung V gerichtete Axialkraft aufnimmt. In Förderrichtung V ist der Lagerkörper 30 gegen den Stützkörper 2 auf Anschlag. Der Lagerkörper 30 lagert auch den Motor 18 und das Getriebe 19 der Fördereinrichtung. Im Ausführungsbeispiel ist er hierfür mit einem seitlichen Fortsatz gebildet, der durch eine seitliche Durchbrechung des Stützkörpers 2 in den seitlichen Aufnahmeraum der Gehäusehüllstruktur 1 ragt, in dem der Motor 18, eine Steuerung und gegebenenfalls ein weitergehendes Gerätemanagementsystem untergebracht sind. Der im Wesentlichen hohlzylindrische Stützkörper 2 ist für diesen Zweck in seinem Mantel mit einer Durchbrechung versehen, durch die auch der Sensor 33 mit einer Sensoranschlussseite ragt, an der er mit der Steuerung und einer Anzeige verbunden ist.

Der Sensor 33 bildet einen elastischen Biegebalken, der an beiden Enden fest eingespannt ist. Der Halterung des Sensors 33 dient ein als einstückig geformter Sensorträger 37, der von dem Stützkörper 2 gleitend axial geführt wird und über eine Kontaktstelle 6 und den Sensor 33 gegen den rückwärtigen Öffnungsrand des Lagerkörpers 30 auf Anschlag oder gegebenenfalls mit dem Lagerkörper 30 fest verbunden ist. Da der Sensorträger 37 auch dann jede Axialbewegung des Lagerkörpers 30 mitmacht, wenn lediglich Anschlagkontakt besteht, wird er dem Lagerkörper 30 und damit der Fördereinrichtung zugerechnet.

Um das Axialspiel zwischen dem Stützkörper 2 und dem Lagerkörper 30 zu eliminieren oder zumindest auf ein Maß zu reduzieren, das in Bezug auf die Dosiergenauigkeit tolerierbar oder nach Möglichkeit in der Praxis nicht mehr spürbar ist, ist ein Kontaktelement 5 vorgesehen, das als weiteres Einstellglied 5 dient. Das Einstellglied 5 bildet die Kontaktstelle 6 für den Sensor 33. Die Kontaktstelle 6 ist ein Nocken, der auf der Translationsachse T von der Vorderseite des Einstellglieds 5 in Förderrichtung V vorragt. Der Lagerkörper 30 ist über den Sensor 33 axial lediglich an der Kontaktstelle 6 quasi punktuell auf der Translationsachse T abgestützt.

Das Einstellglied 5 ist in einem Einstelleingriff mit dem Stützkörper 2. Auch dieser Einstelleingriff ist im Ausführungsbeispiel ein Gewindeeingriff, nämlich zwischen einem Innengewinde 4 an dem rückwärtigen Ende des Stützkörpers 2 und einem entsprechenden Außengewinde des Einstellglieds 5. Das Einstellglied 5 ist eine Kreiszylinderscheibe, die axial gerade so dick ist, dass sie für einen ausreichend festen Einstelleingriff an ihrem äußeren Umfang mit einem ausreichend langen Gewindezug versehen ist.

Die Einstellposition des Einstellglieds 5 ist so gewählt, dass der Lagerkörper 30 gegen den Stützkörper 2 in Förderrichtung auf Anschlag ist und gleichzeitig die Kontaktstelle 6 die Rückseite des Kraftsensors 33 gerade berührt. Das Innengewinde 4 des Stützkörpers 2 ist ausreichend lang, um den Einstellkörper 5 einzuschrauben und im Einstelleingriff bis in diese Einstellposition einstellen zu können. Die Einstellposition ist vorzugsweise so gewählt, dass eine Eichkurve des kalibrierten Sensors 33 nicht verändert wird, insbesondere dass der Nullpunkt der Eichkurve erhalten bleibt. Der Offset des Sensors 33 ist mit anderen Worten daher "Null", wenn der Fluiddruck in dem Behältnis 12 dem Umgebungsdruck entspricht. Ein Offset wird erst bei einem Primen des Infusionsgeräts erhalten. Grundsätzlich kann die Einstellposition aber auch so gewählt sein, dass sich bereits in der Einstellposition ein Offset vor einem Primen einstellt. Dieser Einstelloffset sollte jedoch kleiner sein als der Offset, welcher sich bei einem Primen einstellt. Mit dem Begriff "Primen" wird der Vorgang bezeichnet, durch den die produktführenden Teile bis einschließlich zu einer Austrittsstelle des Katheters 8, der von einer Einstechkanüle oder einer weichen Kanüle gebildet werden kann, vollkommen mit dem Produkt gefüllt sind.

Das Einstellglied 5 ist in seiner Einstellposition an dem Stützkörper 2 festgelegt, vorzugsweise stoffschlüssig mit dem Stützkörper 2 verbunden. Der Stoffschluss kann beispielsweise durch Laserschweißung oder vorzugsweise durch ein in den Einstelleingriff eingebrachtes Adhäsionsmittel erhalten werden.

Auch ohne Okklusions- und/oder Leckerkennung ist ein Axialspiel nicht nur dem Gewindeeingriff zwischen dem Abtriebsglied 10 und dem Antriebsglied 20 inhärent, sondern auch den Drehlagerungen, wie sie von herkömmlichen Infusionsgeräten bekannt sind.

Um das Axialspiel in der Drehlagerung des Antriebsglieds 20 zu reduzieren, ist ein Einstellglied 35 vorgesehen. Das Einstellglied 35 ist in einem Einstelleingriff mit dem Lagerkörper 30. Auch dieser Einstelleingriff ist ein Gewindeeingriff. Wie bereits das Einstellglied 5 ist auch das Einstellglied 35 eine flache, scheibenförmige Schraube mit einem Außengewinde. Das Einstellglied 35 ist an dem rückwärtigen Ende des Lagerkörpers 30 in den Lagerkörper 30 eingeschraubt, der hierfür ein Innengewinde 34 bildet, das mit dem Außengewinde des Einstellkörpers 35 in dem Einstelleingriff ist. Das Einstellglied 35 ist so angeordnet, dass über die von der Drehlagerung ausgehenden Kräfte hinaus keine anderen äußeren Kräfte auf das Einstellglied 35 wirken können.

Für die axiale Abstützung und Festlegung des Antriebsglieds 20 bilden der Lagerkörper 30 eine gegen die Förderrichtung V weisende erste Stützfläche 31 und das Einstellglied 35 eine der Stützfläche 31 zugewandte zweite Stützfläche 32. Das Antriebsglied 20 bildet an seinem Steg 22 eine in die Förderrichtung V weisende dritte Stützfläche 23, die der ersten Stützfläche 31 zugewandt ist, und eine gegen die Förderrichtung V weisende vierte Stützfläche 24, die der zweiten Stützfläche 32 zugewandt ist. Zwischen den beiden Stützflächen 31 und 23 ist ein Kugellager 27 und zwischen den Stützflächen 32 und 24 ist ein weiteres Kugellager 28 axial gehalten. Jedes der Kugellager 27 und 28 bildet ein Radiallager und über die Stützflächen 31 und 23 sowie 32 und 24 ein Axiallager. Die Kugellager weisen wie üblich je einen inneren und einen äußeren Lagerring auf, die um die Translationsachse T relativ zueinander drehbar sind und zwischen denen je eine Mehrzahl von Kugeln angeordnet ist, die die Radial- und Axialkräfte zwischen den Lagerringen übertragen. Bei dem Kugellager 27 ist der innere Lagerring mit 27i und der äußere Lagerring mit 27a bezeichnet. Das Kugellager 28 weist entsprechend einen inneren Lagerring 28i und einen äußeren Lagerring 28a auf. Die inneren Lagerringe 27i und 28i sind radial an der äußeren Umfangsfläche des Antriebsglieds 20 und die äußeren Lagerringe 27a und 28a sind radial an der gegenüberliegenden Mantelinnenfläche des Lagerkörpers 30 abgestützt.

Für die axiale Einspannung der Kugellager 27 und 28 ist das Einstellglied 35 in seiner Einstellposition mit einer geringen axialen Kraft gegen den äußeren Lagerring 28a gepresst. Das Einstellglied 35 und das Kugellager 28 sind nur mit dem äußeren Lagerring 28a und der zweiten Stützfläche 32 in Kontakt. Die zweite Stützfläche 32 ist eine geschlossen umlaufende, zu der Rotationsachse T konzentrische Ringstirnfläche eines Ringstegs 36, der in die Förderrichtung V von der Vorderseite des Einstellglieds 35 aufragt. Die Ringstirnfläche könnte auch Unterbrechungen aufweisen. Ebenso könnte die Stützfläche 32 durch einzeln aufragende Nocken gebildet sein.

Die äußeren Lagerringe 27a und 28a haben axial keinen Kontakt mit den Stützflächen 23 und 24 des Antriebsglieds 20. Die inneren Lagerringe 27i und 28i haben axial keinen Kontakt mit den Stützflächen 31 und 32. Der axiale Kraftfluss verläuft durch die Drehlagerung daher ausschließlich über den Kontakt der Stützflächen 31 und 32 mit dem jeweils zugewandten der äußeren Lagerringe 27a und 28a und den Kontakt zwischen den Stützflächen 23 und 24 und dem jeweils zugewandten der inneren Lagerringe 27i und 28i. Die axiale Kraft wird innerhalb der Kugellager 27 und 28 deshalb ausschließlich durch die Kugeln übertragen. Auf diese Weise entsteht abgesehen von Fertigungstoleranzen der Kugellager 27 und 28 eine axial nahezu spielfreie Drehlagerung.

Das Einstellglied 35 wird bereits wie die zuvor beschriebenen Einstellglieder in seiner Einstellposition gesichert. Die Sicherung an dem Lagerkörper 30 ist ebenfalls vorzugsweise stoffschlüssig und kann insbesondere durch ein Adhäsionsmittel bewirkt werden, das in den Einstelleingriff eingebracht ist. Andere Stoffschlussverbindungen, beispielsweise Laserschweißung, sind jedoch ebenfalls möglich. Wie bei den anderen Einstellgliedern wird die Sicherung im Einstelleingriff selbst vorgenommen.

Zu den Einstelleingriffen ist noch zu bemerken, dass die axialen Längen der Verstellwege der Einstellglieder 5, 15, 25 und 35 in den Einstellgriffen je so lang sind, dass das jeweilige Einstellglied bei der Verstellung in die Einstellposition nicht gegen den Körper, mit dem es in dem Einstelleingriff ist, in einen Anschlagkontakt gelangen kann, der eine weitere Verstellung gleicher Richtung blockiert.

Noch eine weitere Quelle für ein der Dosiergenauigkeit abträgliches Axialspiel ist bei herkömmlichen Infusionsgeräten und auch bei herkömmlichen Injektionsgeräten der große Unterschied zwischen der axialen Wärmeausdehnung der Gehäuse und der axialen Wärmeausdehnung der eingesetzten Reservoirbehältnisse. Die Gehäuse sind üblicherweise aus Kunststoff im Spritzguss hergestellt, während die Behältnisse meist Glaskörper sind. Die Wärmeausdehnungskoeffizienten dieser Materialien unterscheiden sich im Allgemeinen etwa um den Faktor 10, d. h. um eine ganze Größenordnung. Für den axialen Ausgleich dieser Unterschiede in der Wärmeausdehnung sind die Behältnisse bei den herkömmlichen Geräten axial elastisch nachgiebig an den Gehäusen abgestützt. Im Einsatztemperaturbereich der Geräte, der immerhin den Bereich von -20°C bis 40°C abdeckt, ändern sich daher die Positionen zwischen den Fördereinrichtungen und den Behältnissen axial in einem Ausmaß, das die Dosiergenauigkeit spürbar beeinträchtigt.

Diesem Axialspiel und seiner für die Dosiergenauigkeit negativen Wirkung wird dadurch entgegengewirkt, dass der Stützkörper 2 in Axialrichtung eine Wärmeausdehnung aufweist, die deutlich näher bei der axialen Wärmeausdehnung des Behältnisses 12 liegt als die Gehäuse von herkömmlichen Geräten. So kann der Stützkörper 2 insbesondere aus einem Material bestehen, dessen Wärmeausdehnungskoeffizient sich höchstens um einen Faktor 5 von dem Wärmeausdehnungskoeffizient des Materials des Behältnisses 12 unterscheidet. Bevorzugter ist es, wenn die Wärmeausdehnungskoeffizienten so nah als möglich beieinander liegen oder gar gleich sind. Auch konstruktive Maßnahmen sind denkbar, beispielsweise die Fertigung des Stützkörpers 2 als Verbundkörper, der im Verbund mehrere Materialien aufweist, beispielsweise in Kunststoff eingelegte Aussteifungskörper, die die Wärmeausdehnung des Kunststoffmaterials in axialer Richtung behindern. Bevorzugte Materialien für den Erhalt einer günstigen Wärmeausdehnung weisen im Einsatztemperaturbereich einen Wärmeausdehnungskoeffizienten von 30 x 10⁻⁶ / K oder weniger auf. Die Materialien weisen vorzugsweise eine in alle Richtungen gleichmäßige Wärmeausdehnung auf. Eine Stützstruktur in Form eines Verbundkörpers wird allerdings naturgemäß eine ungleichmäßige Wärmeausdehnung, bezogen auf den gesamten Verbundkörper, aufweisen, so dass in solch einem Fall nur die axiale Wärmeausdehnung und der axiale Wärmeausdehnungskoeffizient gemeint sind.

Bevorzugte Konstruktionsmaterialien für Infusionsgeräte und Injektionsgeräte sind zusammen mit deren im Einsatztemperaturbereich geltenden Wärmeausdehnungskoeffizienten *α* in der folgenden Tabelle zusammengestellt:

| Material | Wärmeausdehnungskoeffizient α in 10⁻⁶ / K |
|---|---|
| Messing | 18 bis 19 |
| Stahl | 10 bis 12 |
| Aluminium | 23 bis 24 |
| Polyamid PA | 100 bis 140 |
| Polyoxymethylen POM | 110 bis 130 |
| Polyethylenterephthalat PET | 70 |
| Polycarbonat PC | 70 |
| Polytetrafluorethylen PTFE | 60 bis 200 |
| Acrylnitril/Butadien/Styrol ABS | 80 bis 110 |
| Glas | 5 bis 10 |
| Hartgummi | 75 bis 100 |

Durch einen Stützkörper 2 oder allgemeiner eine Stützstruktur 2 aus beispielsweise Aluminium oder einer Aluminium-Basislegierung kann bereits gegenüber denjenigen Kunststoffmaterialien, die in Bezug auf die Wärmeausdehnung dem Behältnismaterial, vorzugsweise Glas, am nächsten kommen, eine deutliche Verbesserung erzielt werden, da der Wärmeausdehnungskoeffizient von Aluminium um etwa den Faktor 3 kleiner ist als der Wärmeausdehnungskoeffizient der den Behältnismaterial in Bezug auf den Wärmeausdehnungskoeffizient am nächsten kommenden Kunststoffmaterialien. Durch die Verwendung eines Messingmaterials ist eine weitere Verbesserung erzielbar. Wird die Stützstruktur aus Stahl gebildet oder weist sie Stahlkomponenten in solch einer Anordnung auf, dass durch die Stahlkomponenten die axiale Wärmeausdehnung maßgeblich beeinflusst wird, so kann bei entsprechender Wahl des Glasmaterials im günstigsten Fall sogar eine identische Wärmeausdehnung erzielt werden. Wird der Stützkörper 2 oder allgemeiner eine Stützstruktur 2, die natürlich ebenfalls axiale Stützfunktion wie der Stützkörper 2 übernimmt, als Verbundkörper gebildet, so kann durch Aussteifungskörper, beispielsweise in eine Kunststoffmatrix eingelagerte Axialfasern, ein vergleichbar günstiges Wärmeausdehnungsverhalten erzielt werden, wenn der oder die Aussteifungskörper eine Wärmeausdehnung wie vorstehend beschrieben aufweisen.

Auf die Mehrteiligkeit des Gehäuses, im Ausführungsbeispiel Zweiteiligkeit, kann grundsätzlich sogar verzichtet werden, wenn die Gehäusehülle, im Ausführungsbeispiel die Hüllstruktur 1, eine erfindungsgemäße Wärmeausdehnung besitzt. Bei solch einer Ausbildung einer Gehäusehülle wird es bevorzugt, wenn die Gehäusehülle als Verbundkörper gebildet ist, beispielsweise als Kunststoffmatrix mit eingelegten Aussteifungskörpern wie insbesondere axial orientierte Metallfasern.

Wenngleich bereits allein eine Stützstruktur vorteilhaft ist, die nur das Behältnis axial abstützt, ist es vorteilhafter, wenn solch eine Stützstruktur sich über eine möglichst große, in Förderrichtung V des Kolbens 13 gemessene Länge erstreckt. Die Stützstruktur sollte, wie beispielhaft der Stützkörper 2, zusätzlich auch noch die Fördereinrichtung axial in beide Richtungen abstützen. Besonders günstig ist es ferner, wenn auch die Fördereinrichtung im Ganzen eine axiale Wärmeausdehnung aufweist, die möglichst nah bei der axialen Wärmeausdehnung der Stützstruktur angesiedelt ist, beispielsweise indem die Stützstruktur und die Komponenten der Fördereinrichtung aus dem gleichen Material oder aus gegebenenfalls unterschiedlichen Materialien mit möglichst nahe beieinander liegender axialer Wärmeausdehnung gefertigt sind. Vorteilhafterweise weist das Abtriebsglied 10 oder das Antriebsglied 20 oder weisen vorzugsweise diese beiden Komponenten je eine im wesentlichen gleiche axiale Wärmeausdehnung wie der Stützkörper 2 auf, d.h. eine Wärmeausdehnung, die sich um höchstens den Faktor 5 und bevorzugter um weniger als den Faktor 5, möglichst um höchstens den Faktor 2 oder noch weniger von der axialen Wärmeausdehnung des Stützkörpers 2 unterscheidet und idealerweise gleich ist.

Je größer die von der bevorzugt einteiligen Stützstruktur oder den mehreren Stützkörpern einer mehrteiligen Stützstruktur erst gemeinsam überbrückte axiale Länge ist, desto geringer ist das auf unterschiedliche axiale Wärmeausdehnungen zurückzuführende Axialspiel. Kunststoffteile herkömmlicher Art dürfen hierbei durchaus kurze axiale Längen überbrücken. Je kürzer die von herkömmlichen Kunststoffteilen überbrückten axialen Längen sind, desto geringer ist das auf unterschiedliche Wärmeausdehnungen zurückzuführende Axialspiel. Besonders günstig ist es, wie im Ausführungsbeispiel, wenn solch ein Stützkörper oder gegebenenfalls auch mehrere axial hintereinander angeordnete Stützkörper vorgesehen ist oder sind, der oder die bezüglich ihrer axialen Wärmeausdehnung nahe bei derjenigen des Behältnisses und/oder der Fördereinrichtung liegen. Die Stützeinrichtungen des Stützkörpers, die das Behältnis und/oder die Fördereinrichtung axial an dem Stützkörper oder den Stützkörpern festlegen, sollten von jeweiligen dem Stützkörper in einem Stück gebildet oder mit dem jeweiligen Stützkörper so verbunden sein, dass sie relativ zu dem Stützkörper axial nicht bewegbar sind, wie beispielsweise das Paar der Stege 3 und 7a, das Paar aus Stützsteg 3 und Einstellglied 5 und das Paar aus Einstellglied 35 und Stützfläche 31.

Der Stützkörper 2 ist ein vergleichsweise einfacher Hülsenkörper, der in die Hüllstruktur 1 eingesetzt ist und lediglich der Lagerung der relativ zueinander axial bewegbaren Teile und damit zur axialen Aussteifung vorgesehen ist. Die Hüllstruktur 1 selbst kann wie üblich aus Kunststoff im Spritzguss hergestellt sein. Die Hüllstruktur 1 besteht aus zwei Teilen, nämlich einem Hauptteil und einem Bodenteil. Das Hauptteil bildet die Aufnahmekammer für die Stützstruktur 2 und die gegebenenfalls nicht von der Stützstruktur 2 gelagerten Komponenten der Verabreichungsvorrichtung. Das Bodenteil ist eine einfache Platte, die mit dem Hauptteil an dessen Rückseite fest verbunden ist und dort die Aufnahmekammer verschließt.

Der Deckel 7 besteht vorzugsweise aus dem gleichen Material wie der Stützkörper 2. Ebenso gilt dies für den Lagerkörper 30, die beiden Einstellglieder 35 und 5 und die Trägerscheibe 37, so dass eine insgesamt in Bezug auf die axiale Wärmesausdehnung sehr homogene Stützstruktur erhalten wird. Eine Fertigung des Deckels 7 und/oder der Trägerscheibe 37 und/oder des Einstellglieds 35 und/oder des Einstellglieds 5 aus einem der üblichen Kunststoffmaterialien ist jedoch weit weniger kritisch als die Bildung einer axial langen Stützstruktur aus den herkömmlichen Kunststoffmaterialien.

Figur 6 zeigt in einem Längsschnitt einen wie im ersten Ausführungsbeispiel gelagerten Lagerkörper 30 mit den von ihm gelagerten Komponenten einer Verabreichungsvorrichtung nach einem zweiten Ausführungsbeispiel, die ebenfalls ein Infusionsgerät ist. Diejenigen Komponenten des zweiten Ausführungsbeispiels, die ihrer Funktion und zum Teil auch Konstruktion nach mit Komponenten des ersten Ausführungsbeispiels vergleichbar sind, tragen die gleichen Bezugszeichen wie im ersten Ausführungsbeispiel. Unterschiede bestehen nur soweit nachfolgend hierauf aufmerksam gemacht wird oder aus den Figuren selbst sich ergibt. Es sollen die Ausführungen zum ersten Ausführungsbeispiel gelten, soweit sich hieraus keine offensichtlichen Widersprüche ergeben.

Die Verabreichungsvorrichtung des zweiten Ausführungsbeispiels weist eine Spielreduziereinrichtung lediglich für die Eliminierung oder zumindest Reduzierung des Axialspiels zwischen dem Rotationsglied 20 und dem Lagerkörper 30 auf. Die Spielreduziereinrichtung spannt das Rotationsglied 20 im Unterschied zum ersten Ausführungsbeispiel axial unmittelbar gegen den Sensorträger 37. Ferner umgibt in dem zweiten Ausführungsbeispiel das Translationsglied 10 das Rotationsglied 20. Das Translationsglied 10 und das Rotationsglied 20 sind miteinander in einem Gewindeeingriff. Hierfür ist das Rotationsglied 20 über den größten Teil seiner axialen Länge mit einem Außengewinde 21 und das Translationsglied 10 nur an seinem bezüglich der Translationsrichtung V rückwärtigen Ende mit einem Innengewinde 11 versehen. Das Translationsglied 10 ist an dem Lagerkörper 30 axial linear geführt. Wie bei dem ersten Ausführungsbeispiel treibt ein Motor 18, vorzugsweise ein elektrischer Schrittmotor, über ein Stirnradgetriebe mit zwei in Stirneingriff befindlichen Zahnrädern 19 das Rotationsglied 20 um die Rotations- und Translationsachse T rotatorisch an. Das Rotationsglied 20 ist für seinen Antrieb an seinem rückwärtigen Ende wieder mit einem umlaufenden außenverzahnten Ringsteg 22 versehen, der mit dem Zwischenrad 19 des Stirnradgetriebes für den rotatorischen Antrieb des Rotationsglieds 20 in einem Stirneingriff ist.

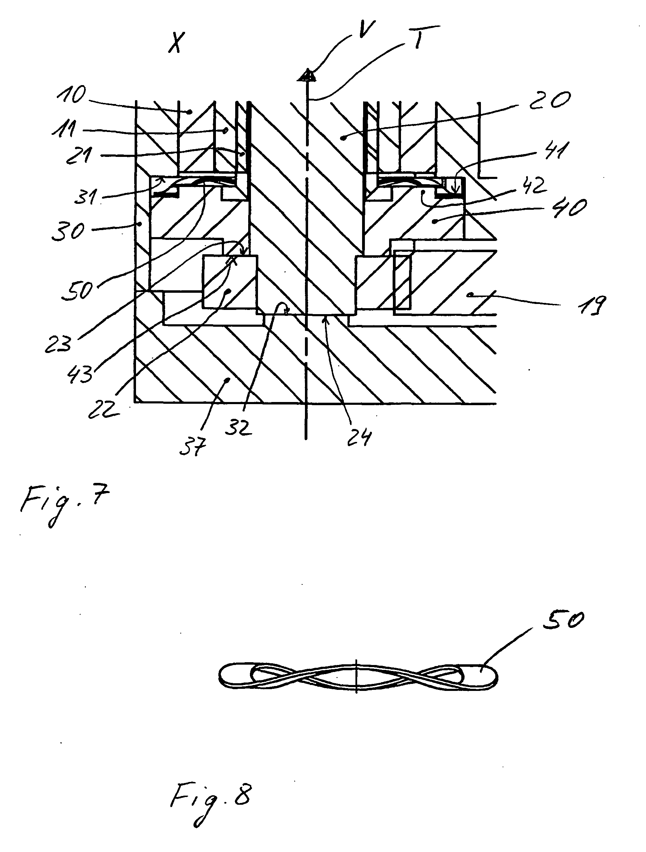

Die Drehlagerung des Rotationsglieds 20 ist in Figur 7 vergrößert dargestellt. Die Drehlagerung des zweiten Ausführungsbeispiels ist als einfache Gleitlagerung gebildet.

Die zweite Stützfläche 32 des Sensorträgers 37 und die vierte Stützfläche 24 des Rotationsglieds 20 bilden ein erstes Gleitpaar der Drehlagerung. Die beiden Stützflächen 32 und 24 befinden sich unmittelbar miteinander in Gleitkontakt. Die zweite Stützfläche 32 ist an dem rückwärtigen Stirnende des Rotationsglieds 20 gebildet. Von dem Sensorträger 37 ragt ihr entgegen ein kurzer Sockel auf, dessen Stirnfläche die zweite Stützfläche 24 bildet. Der Sockel stellt das Rotationsglied 20 von dem Sensorträger 37 frei. Die Formung eines Sockels ermöglicht eine präzisere Fertigung der zweiten Stützfläche 32. Die dritte Stützfläche 23 ist in der Art wie die Stützfläche 23 des ersten Ausführungsbeispiels gebildet, nämlich durch die in Translationsrichtung T weisende vordere Stirnfläche des Ringstegs 22. Die ihr axial zugewandte erste Stützfläche 31 wird von dem Lagerkörper 30 gebildet. Allerdings sind die Stützflächen 31 und 23 radial zueinander versetzt, d.h. sie liegen axial nicht exakt in der Flucht. Der Radialversatz wird von einem Übertragungskörper 40, im Ausführungsbeispiel ein Übertragungsring, überbrückt, der zwischen den Stützflächen 31 und 23 angeordnet ist. Der Übertragungskörper 40 bildet an einer vorderen Stirnseite der ersten Stützfläche 31 axial in der Flucht gegenüberliegend eine um die Translationsachse T und das Rotationsglied 20 umlaufende vordere Stützfläche 41 und an seiner Rückseite der dritten Stützfläche 23 axial in der Flucht gegenüberliegend eine rückwärtige Stützfläche 43. Die rückwärtige Stützfläche 43 ist unmittelbar auf Anschlagkontakt mit der dritten Stützfläche 23. Zwischen der ersten Stützfläche 31 und der ihr zugewandten vorderen Stützfläche 41 verbleibt ein lichter, axialer Abstand. Zwischen den beiden Stützflächen 31 und 41 ist eine Ringfeder 50 angeordnet, die sich axial an beiden Stützflächen 31 und 41 mit einer axialen Vorspannkraft abstützt.

Die Ringfeder 50 ist in Figur 8 einzeln dargestellt. Sie ist umlaufend wellenförmig und beispielsweise aus Federstahl gefertigt. Im eingebauten Zustand stützt sie sich mit ihren Wellenbergen und Wellentälern umlaufend alternierend an der ersten Stützfläche 31 und der vorderen Stützfläche 41 des Übertragungskörpers 40 ab. Bei axialer Stauchung wirkt sie wie eine Blattfeder.

Wie insbesondere in Figur 7 erkennbar, dient der Übertragungskörper 40 nicht nur dem Ausgleich des Radialversatzes, sondern auch der Zentrierung der Ringfeder 40. Hierfür ist der Übertragungskörper 40 an seiner vorderen Stirnseite mit einem umlaufenden Vorsprung 42 versehen, um dessen äußeren Umfang die vordere Stützfläche 41 axial ein Stück weit zurückgenommen umläuft.

Die Ringfeder 50 und der Übertragungskörper 40 bilden die Spielreduziereinrichtung des zweiten Ausfiihrungsbeispiels, indem der Übertragungskörper 40 relativ zu dem Lagerkörper 30 axial bewegbar ist. Vorzugsweise wird er von dem Lagerkörper 30 axial linear geführt. Grundsätzlich kann er jedoch relativ zu dem Lagerkörper 30 drehbeweglich sein. Obgleich der Übertragungskörper 40 grundsätzlich mit dem Rotationsglied 20 drehsteif verbunden sein kann, entweder mittels Fügeverbindung oder durch eine einstückige Ausbildung mit dem Rotationsglied 20, wird es bevorzugt, wenn der Übertragungskörper 40 wie im Ausführungsbeispiel relativ zu dem Rotationsglied 20 drehbeweglich und noch bevorzugter auch axial beweglich angeordnet ist. Auf diese Weise wird ein weiteres Gleitflächenpaar der Drehlagerung durch die unmittelbar aneinander abgleitenden Stützflächen 23 und 43 gebildet. Die Ringfeder 50 wird somit vorteilhafterweise von Drehbewegungen freigehalten.

Im zweiten Ausführungsbeispiel wird im Ergebnis eine vorteilhaft einfache Spielreduziereinrichtung für das Rotationsglied 20 erhalten, die bei ausreichender Vorspannung der Ringfeder 50 jegliches Axialspiel zwischen dem Rotationsglied 20 und dem Lagerkörper 30 eliminiert. In Ausführungen, in denen die Stützflächen 31 und 23 axial einander in der Flucht gegenüberliegen, könnte auf den Übertragungskörper 40 verzichtet werden. Um die Ringfeder 50 jedoch auch in solchen Ausführungen von Drehbewegungen freizuhalten und/oder eine einfach fertigbare Zentrierung für die Ringfeder 50 oder auch eine die Presskraft erzeugende andere Federeinrichtung zu erhalten, ist die Zwischenschaltung eines Übertragungskörpers in der Art des Übertragungskörpers 40 auch dann von Vorteil.

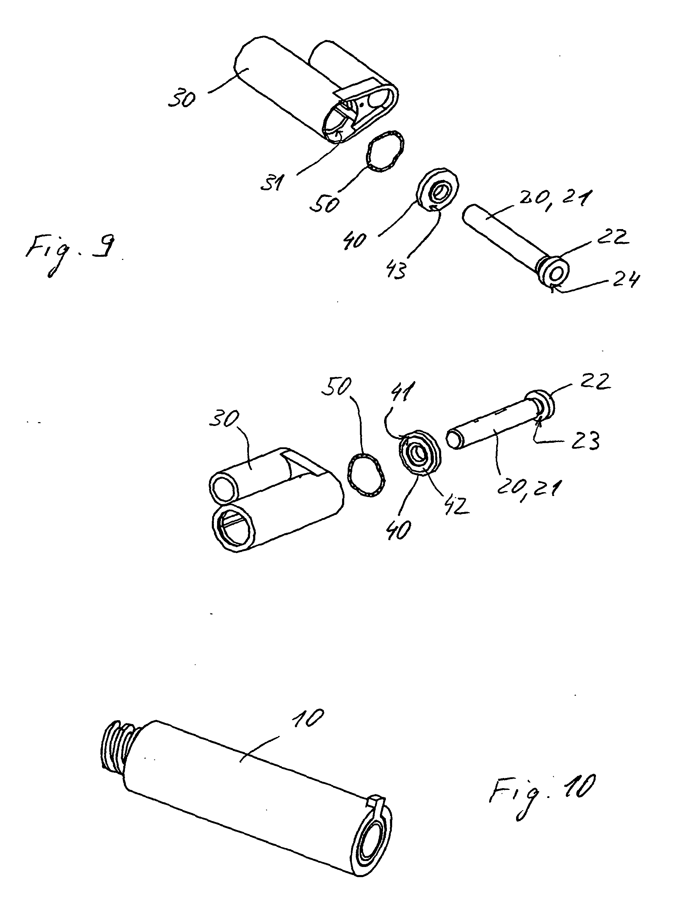

Figur 9 zeigt in einer Explosionsdarstellung den Lagerkörper 30, das Rotationsglied 20, den Übertragungskörper 40 und die Ringfeder 50 entlang der zu denkenden Translationsachse in einer für die Montage geeigneten Folge aufgereiht. Figur 10 zeigt das Translationsglied 10 einzeln. Bei der Montage kann in einem ersten Schritt das Translationsglied 10 alleine von hinten in den Lagerkörper eingesetzt und anschließend das Rotationsglied 20 in das Translationsglied 10 eingeschraubt werden, oder es kann zuerst die Gewindeverbindung zwischen dem Translationsglied 10 und dem Rotationsglied 20 hergestellt und erst anschließend das Translationsglied 10 mit dem eingeschraubten Rotationsglied 20 in den Lagerkörper 30 eingesetzt werden. Vor dem Einschrauben des Rotationsglieds 20 werden der Übertragungskörper 40 und die Ringfeder 50 über das Außengewinde 21 bis gegen den Ringsteg 22 des Rotationsglieds 20 geschoben, und anschließend wird das Rotationsglied 20 in das Translationsglied 10 eingeschraubt. Nachdem das Translationsglied 10 und das Rotationsglied 20 in dem Lagerkörper 30 angeordnet sind, wird der Sensorträger 37 mit dem in Figur 9 gezeigten Hauptteil des Lagerkörpers 30 verbunden, so dass es die zum Zwecke der Montage offene Rückseite des Lagerkörpers 30 verschließt. Lagerkörper 30 und Sensorträger 37 sind im verbundenen Zustand relativ zueinander nicht bewegbar. Die Verbindung ist ferner so gestaltet, dass die Ringfeder 50 mit einer definierten axialen Vorspannkraft eingebaut ist.

Der Gewindeeingriff der Gewinde 11 und 21 ist im zweiten Ausführungsbeispiel als einfacher Gewindeeingriff gebildet, kann jedoch ohne weiteres wie bei dem ersten Ausführungsbeispiel mittels einer zusätzlich Spielreduziereinrichtung axialspielreduzierend gebildet sein.

Schließlich sei auch darauf hingewiesen, dass bei dem ersten Ausführungsbeispiel eine Spielreduziereinrichtung für die Reduzierung oder vorzugsweise Eliminierung von Axialspiel der Drehlagerung des Rotationsglieds 20 wie bei dem zweiten Ausführungsbeispiel gebildet sein kann und dass umgekehrt die auf dem verstellbaren Einstelleingriff beruhende Spielreduziereinrichtung 35 beim zweiten Ausführungsbeispiel statt der Spielreduziereinrichtung 40, 50 vorgesehen sein kann. Auch Mischformen sind denkbar. So könnte eines der Wälzlager 27 und 28 zwischen einem der Stützflächenpaare 31, 23 und 32, 24 oder je solch ein Wälzlager zwischen beiden Stützflächenpaaren angeordnet sein, wobei das oder die beiden Wälzlager vorzugsweise je wie die Wälzlager 27 und 28 des ersten Ausführungsbeispiels angeordnet wären, d.h. es würde die Ringfeder 50 oder eine alternative Feder nur auf eine der Lagerschalen solch eines Wälzlagers wirken.

Der Lagerkörper 30 kann unter Verzicht auf die axial bewegliche Lagerung und den Sensor 33 zu einem Gehäuse mit einer Aufnahme für ein Reservoir 12 abgewandelt werden und dann direkt als Hüllstruktur wie die Hüllstruktur des ersten Ausführungsbeispiels dienen. Solch eine Hüllstruktur kann wie herkömmliche Gehäuse gebildet sein. Alternativ kann sie jedoch die Wärmeausdehnungseigenschaften der Stützstruktur 2 des ersten Ausführungsbeispiels haben, so dass auf die diesbezüglichen Ausführungen zum ersten Ausführungsbeispiel verwiesen wird.

### Bezugszeichen:

- 1: Gehäusestruktur, Hüllstruktur
- 2: Gehäusestruktur, Stützstruktur, Stützkörper
- 3: Stützschulter, Stützsteg
- 4: Gewinde
- 5: Einstellglied, Kontaktelement
- 6: Kontaktstelle
- 7: Abschlusselement, Deckel
- 7a: Gegenstützschulter, Gegenstützsteg
- 8: Katheter
- 9: Eingriffseinrichtung, Gewinde
- 10: Abtriebsglied, Translationsglied
- 11: Gewinde
- 11f: Eingriffsflanke
- 12: Reservoir, Behältnis
- 13: Kolben
- 14: Auslass
- 15: Einstellglied
- 16: Eingriffsglied
- 17: Rückstellelement
- 18: Motor
- 19: Zahnräder
- 20: Antriebsglied, Rotationsglied
- 21: Gewinde
- 21f: Eingriffsflanke
- 22: Steg
- 23: Stützfläche
- 24: Stützfläche
- 25: Einstellglied
- 25f: Eingriffsflanke
- 26: Gewinde
- 27: Wälzlager
- 27a: äußerer Lagerring
- 27i: innerer Lagerring
- 28: Wälzlager
- 28a: äußerer Lagerring
- 28i: innerer Lagerring
- 29: Führungsbahn
- 30: Lagerkörper
- 31: Stützfläche
- 32: Stützfläche
- 33: Sensor
- 34: Gewinde
- 35: Einstellglied
- 36: Steg
- 37: Sensorträger
- 38: -
- 39: -
- 40: Übertragungskörper
- 41: vordere Stützfläche
- 42: Vorsprung
- 43: rückwärtige Stützfläche
- 44-49: -
- 50: Feder

- T: Translationsachse, Rotationsachse
- V: Förderrichtung

## Patentansprüche

1. Vorrichtung für die dosierte Verabreichung eines injizierbaren Produkts, die Vorrichtung umfassend:
a) ein Gehäuse (1, 2), das ein Produktreservoir oder eine Aufnahme für ein Produktreservoir (12) bildet,
b) ein Translationsglied (10), das für die Förderung des Produkts eine Translationsbewegung in eine Translationsrichtung (V) ausführt,
c) ein Rotationsglied (20), das für die Förderung eine Rotationsbewegung um eine Rotationsachse (T) ausführt und mit dem Translationsglied (10) so gekoppelt ist, dass es das Translationsglied (10) gegen die Translationsrichtung stützt und eine der Bewegungen aus Rotationsbewegung und Translationsbewegung die andere bewirkt,
d) eine Drehlagerung, die einen von dem Gehäuse (1, 2) gebildeten oder axial abgestützten Lagerkörper (30; 1) umfasst, der das Rotationsglied (20) um die Rotationsachse (T) drehbar lagert, und die eine erste Stützfläche (31), eine zweite Stützfläche (32), eine dritte Stützfläche (23) und eine vierte Stützfläche (24) aufweist, um das Rotationsglied (20) axial an dem Lagerkörper (30; 1) zu sichern,
**dadurch gekennzeichnet, dass**
e) die erste Stützfläche (31) und die zweite Stützfläche (32) axial steif mit dem Lagerkörper (30; 1) verbunden und entweder voneinander axial abgewandt oder einander axial zugewandt sind und wobei die dritte Stützfläche (23) der ersten Stützfläche (31) und die vierte Stützfläche (24) der zweiten Stützfläche (32) axial zugewandt und mit dem Rotationsglied (20) axial steif verbunde sind,
f) und dass die Vorrichtung eine Spielreduziereinrichtung (35; 40, 50) umfasst, die wenigstens zwei der Stützflächen (31, 32, 23, 24), die einander axial zugewandt sind, mit einer Presskraft axial aufeinander zu spannt und dadurch ein Axialspiel der Drehlagerung eliminiert oder reduziert.

2. Vorrichtung nach Anspruch 1, **dadurch gekennzeichnet, dass** die Spielreduziereinrichtung (40, 50) die Presskraft als eine Elastizitätskraft erzeugt.

3. Vorrichtung nach einem der vorhergehenden Ansprüche, **dadurch gekennzeichnet, dass** die Spielreduziereinrichtung (40, 50) eine die Presskraft erzeugende Feder (40) umfasst.

4. Vorrichtung nach dem vorhergehenden Anspruch, **dadurch gekennzeichnet, dass** die Feder (50) wie eine Blattfeder wirkt.

5. Vorrichtung nach einem der zwei vorhergehenden Ansprüche, **dadurch gekennzeichnet, dass** die Feder (50) ein das Rotationsglied (20) umgebender, axial elastischer Ringkörper ist.

6. Vorrichtung nach dem vorhergehenden Anspruch, **dadurch gekennzeichnet, dass** der Ringkörper (50) umlaufend gewellt ist.

7. Vorrichtung nach einem der vorhergehenden Ansprüche, **dadurch gekennzeichnet, dass** die Spielreduziereinrichtung (40, 50) einen relativ zu dem Gehäuse (1) oder Lagerkörper (30) axial beweglichen Übertragungskörper (40) umfasst, der zwischen zwei (31, 23) der axial einander zugewandten Stützflächen (31, 23, 32, 24) angeordnet ist und die axiale Presskraft zwischen diesen Stützflächen (31, 23) überträgt.

8. Vorrichtung nach dem vorhergehenden Anspruch, **dadurch gekennzeichnet, dass** der Übertragungskörper (40) von dem Lagerkörper (30) oder Gehäuse (1) axial und optional auch verdrehgesichert geführt wird.

9. Vorrichtung nach einem der zwei vorhergehenden Ansprüche, **dadurch gekennzeichnet, dass** die Stützflächen (31, 23), zwischen denen der Übertragungskörper (40) angeordnet ist, radial zueinander versetzt sind und der Übertragungskörper (40) diesen Radialversatz überbrückt.

10. Vorrichtung nach einem der vorhergehenden Ansprüche, **dadurch gekennzeichnet, dass** ein Bodenteil (37), vorzugsweise ein Sensorträger (37), des Lagerkörpers (1) oder des Gehäuses (1) einen axial abragenden Sockel aufweist, der an seinem Stirnende die zweite Stützfläche (32) bildet.

11. Vorrichtung nach dem vorhergehenden Anspruch, **dadurch gekennzeichnet, dass** das Rotationsglied (20) drehend auf dem Sockel gleitet.

12. Vorrichtung nach einem der vorhergehenden Ansprüche, **dadurch gekennzeichnet, dass** das Rotationsglied (20) einen Ringsteg (22) aufweist, der wenigstens eine der Stützflächen (23, 24) des Rotationsglieds (20) bildet.

13. Vorrichtung nach einem der vorhergehenden Ansprüche, **dadurch gekennzeichnet, dass** die Spielreduziereinrichtung (35) die erste oder die zweite Stützfläche (32) bildet und mit dem Lagerkörper (30) in einem Einstelleingriff ist, in dem die erste Stützfläche (31) und die zweite Stützfläche (32) relativ zueinander in solch eine Einstellposition verstellt und in der Einstellposition relativ zueinander axial gesichert sind, dass ein Axialspiel der Drehlagerung reduziert wird.

14. Vorrichtung nach einem der vorhergehenden Ansprüche, **dadurch gekennzeichnet, dass** die Spielreduziereinrichtung (35) die dritte oder die vierte Stützfläche bildet und mit dem Rotationsglied in einem Einstelleingriff ist, in dem die dritte Stützfläche und die vierte Stützfläche relativ zueinander in solch eine Einstellposition verstellt und in der Einstellposition relativ zueinander axial gesichert sind, dass ein Axialspiel der Drehlagerung reduziert wird.

15. Vorrichtung nach einem der vorhergehenden Ansprüche, **dadurch gekennzeichnet, dass** zwischen der ersten Stützfläche (31) und der dritten Stützfläche (23) ein Wälzlager (27) mit einem radial inneren Lagerring (27i) und einem radial äußeren Lagerring (27a) angeordnet ist.

16. Vorrichtung nach dem vorhergehenden Anspruch, **dadurch gekennzeichnet, dass** einer der Lagerringe (27i, 27a) von der ersten Stützfläche (31), aber nicht von der dritten Stützfläche (23) axial gestützt wird und der andere der Lagerringe (27i, 28a) von der dritten Stützfläche (23), aber nicht von der ersten Stützfläche (31) axial gestützt wird.

17. Vorrichtung nach einem der vorhergehenden Ansprüche, **dadurch gekennzeichnet, dass** zwischen der zweiten Stützfläche (32) und der vierten Stützfläche (24) ein Wälzlager (28) mit einem radial inneren Lagerring (28i) und einem radial äußeren Lagerring (28a) angeordnet ist.

18. Vorrichtung nach dem vorhergehenden Anspruch, **dadurch gekennzeichnet, dass** einer der Lagerringe (28i, 28a) von der zweiten Stützfläche (32), aber nicht von der vierten Stützfläche (24) axial gestützt wird und der andere der Lagerringe (28i, 28a) axial von der vierten Stützfläche (24), aber nicht von der zweiten Stützfläche (32) gestützt wird.

19. Vorrichtung nach einem der vorhergehenden Ansprüche, **dadurch gekennzeichnet, dass** die Drehlagerung eine Wälzlagerung ist, die zwei Wälzlager (27, 28) umfasst, und dass eines der Wälzlager (27, 28) zwischen der ersten Stützfläche (31) und der dritten Stützfläche (23) und das andere der Wälzlager (27, 28) zwischen der zweiten Stützfläche (32) und der vierten Stützfläche (24) angeordnet ist, wobei die Stützfläche (32) der Spielreduziereinrichtung (35) so gebildet ist, dass sie im Einstelleingriff der Spielreduziereinrichtung (35) Kontakt weder mit dem Rotationsglied (20) noch mit dem Lagerkörper (30), sondern nur mit einem der Wälzlager (27, 28) haben kann.

20. Vorrichtung nach dem vorhergehenden Anspruch, **dadurch gekennzeichnet, dass** die Stützfläche (32) der Spielreduziereinrichtung (35) so gebildet ist, dass sie in dem Einstelleingriff der Spielreduziereinrichtung (35) Kontakt nur mit einem einzigen der Lagerringe des einen der Wälzlager (27, 28) haben kann.

21. Vorrichtung nach einem der vorhergehenden Ansprüche, **dadurch gekennzeichnet, dass** die Spielreduziereinrichtung (35) in der Einstellposition durch Stoffschluss entweder mit dem Lagerkörper (30) oder dem Rotationsglied (20) verbunden und dadurch gesichert ist.

22. Vorrichtung nach einem der vorhergehenden Ansprüche, **dadurch gekennzeichnet, dass** ein axial in sich steifes Einstellglied (35) die Spielreduziereinrichtung bildet.

23. Vorrichtung nach einem der vorhergehenden Ansprüche, **dadurch gekennzeichnet, dass** das Rotationsglied (20) die dritte Stützfläche (23) und die vierte Stützfläche (24) in einem Stück bildet.

24. Vorrichtung nach einem der vorhergehenden Ansprüche, **dadurch gekennzeichnet, dass** die Spielreduziereinrichtung (35) mit dem Lagerkörper (30) in dem Einstelleingriff ist.

25. Vorrichtung nach einem der vorhergehenden Ansprüche, **dadurch gekennzeichnet, dass** der Einstelleingriff auf Form- und Kraftschluss beruht.

26. Vorrichtung nach dem vorhergehenden Anspruch, **dadurch gekennzeichnet, dass** der Einstelleingriff ein Gewindeeingriff ist.

27. Vorrichtung nach dem vorhergehenden Anspruch, **dadurch gekennzeichnet, dass** der Lagerkörper (30) ein Hülsenkörper ist, der das Rotationsglied (20) umgibt und ein Innengewinde (34) aufweist, das mit einem Außengewinde der Spielreduziereinrichtung (35) in dem Gewindeeingriff ist.

28. Vorrichtung nach einem der vorhergehenden Ansprüche, **dadurch gekennzeichnet, dass** das Translationsglied (10) und das Rotationsglied (20) je wenigstens eine Eingriffsflanke (11f, 21f) bilden und mittels der Eingriffsflanken (11f, 21f) in solch einem Flankeneingriff sind, dass die Rotationsbewegung des Rotationsglieds (20) die Translationsbewegung des Translationsglieds (10) bewirkt, und dass eine weitere Spielreduziereinrichtung (25; 15, 17) vorgesehen ist, die in einem Einstelleingriff mit dem Translationsglied (10) und dem Rotationsglied (20) relativ zu dem Translationsglied (10) und dem Rotationsglied (20) in solch eine Einstellposition verstellt und in der Einstellposition so gesichert ist, dass ein Axialspiel des Flankeneingriffs reduziert ist.

29. Vorrichtung nach dem vorhergehenden Anspruch, **dadurch gekennzeichnet, dass** der Flankeneingriff ein Gewindeeingriff ist.

30. Vorrichtung nach einem der zwei vorhergehenden Ansprüche, **dadurch gekennzeichnet, dass** der Einstelleingriff der weiteren Spielreduziereinrichtung (25; 15, 17) mit dem Translationsglied (10) ein Gewindeeingriff ist.

31. Vorrichtung nach einem der drei vorhergehenden Ansprüche, **dadurch gekennzeichnet, dass** der Einstelleingriff der weiteren Spielreduziereinrichtung (25) mit dem Rotationsglied (20) ein Gewindeeingriff ist.

## Claims

1. A device for administering an injectable product in doses, said device comprising:
a) a housing (1, 2) which forms a product reservoir or a receptacle for a product reservoir (12);
b) a translational member (10) which performs a translational movement in a translational direction (V) in order to deliver the product;
c) a rotational member (20) which, for delivering the product, performs a rotational movement about a rotational axis (T) and is coupled to the translational member (10) such that it supports the translational member (10) counter to the translational direction and such that one of the rotational movement and translational movement generates the other of the rotational movement and translational movement;
d) a rotary bearing comprising a bearing body (30; 1) which is formed or axially supported by the housing (1, 2) and mounts the rotational member (20) such that it can rotate about the rotational axis (T), and comprising a first support surface (31), a second support surface (32), a third support surface (23) and a fourth support surface (24), in order to secure the rotational member (20) axially on the bearing body (30; 1);
**characterised in that**
e) the first support surface (31) and the second support surface (32) are connected, axially rigid, to the bearing body (30; 1) and either face away from each other axially or face each other axially, and **in that** the third support surface (23) which axially faces the first support surface (31), and the fourth support surface (24) which axially faces the second support surface (32), are connected, axially rigid, to the rotational member (20);
f) and **in that** the device comprises a play reducing means (35; 40, 50) by which at least two of the support surfaces (31, 32, 23, 24) which axially face each other are clamped axially towards each other with a pressing force, so as to eliminate or reduce an axial play in the rotary bearing.

2. The device according to claim 1, **characterised in that** the play reducing means (40, 50) generates the pressing force as an elasticity force.

3. The device according to any one of the preceding claims, **characterised in that** the play reducing means (40, 50) comprises a spring (50) which generates the pressing force.

4. The device according to the preceding claim, **characterised in that** the spring (50) acts like a leaf spring.

5. The device according to any one of the preceding two claims, **characterised in that** the spring (50) is an axially elastic annular body which surrounds the rotational member (20).

6. The device according to the preceding claim, **characterised in that** the annular body (50) is circumferentially undulated.

7. The device according to any one of the preceding claims, **characterised in that** the play reducing means (40, 50) comprises a transmission body (40) which: can be axially moved relative to the housing (1) or the bearing body (30); is arranged between two (31, 23) of the support surfaces (31, 23, 32, 24) which axially face each other; and transmits the axial pressing force between these support surfaces (31, 23).

8. The device according to the preceding claim, **characterised in that** the transmission body (40) is guided axially, and optionally also secured against twisting, by the bearing body (30) or the housing (1).

9. The device according to any one of the preceding two claims, **characterised in that** the support surfaces (31, 23) between which the transmission body (40) is arranged are radially offset with respect to each other, and the transmission body (40) spans this radial offset.

10. The device according to any one of the preceding claims, **characterised in that** a base part (37), preferably a sensor carrier (37), of the bearing body (30) or the housing (1) comprises an axially protruding pedestal which forms the second support surface (32) at its facing end.

11. The device according to the preceding claim, **characterised in that** the rotational member (20) slides in rotation on the pedestal.

12. The device according to any one of the preceding claims, **characterised in that** the rotational member (20) comprises an annular web (22) which forms at least one of the support surfaces (23, 24) of the rotational member (20).

13. The device according to any one of the preceding claims, **characterised in that** the play reducing means (35) forms the first or second support surface (32) and is in an adjusting engagement with the bearing body (30) in which the first support surface (31) and the second support surface (32) are displaced relative to each other into an adjusting position and are axially secured relative to each other in the adjusting position such that an axial play in the rotary bearing is reduced.

14. The device according to any one of the preceding claims, **characterised in that** the play reducing means (35) forms the third or fourth support surface and is in an adjusting engagement with the rotational member in which the third support surface and the fourth support surface are displaced relative to each other into an adjusting position and are axially secured relative to each other in the adjusting position such that an axial play in the rotary bearing is reduced.

15. The device according to any one of the preceding claims, **characterised in that** a roller bearing (27) comprising a radially inner bearing ring (27i) and a radially outer bearing ring (27a) is arranged between the first support surface (31) and the third support surface (23).

16. The device according to the preceding claim, **characterised in that** one of the bearing rings (27i, 27a) is axially supported by the first support surface (31) but not by the third support surface (23), and the other of the bearing rings (27i, 27a) is axially supported by the third support surface (23) but not by the first support surface (31).

17. The device according to any one of the preceding claims, **characterised in that** a roller bearing (28) comprising a radially inner bearing ring (28i) and a radially outer bearing ring (28a) is arranged between the second support surface (32) and the fourth support surface (24).

18. The device according to the preceding claim, **characterised in that** one of the bearing rings (28i, 28a) is axially supported by the second support surface (32) but not by the fourth support surface (24), and the other of the bearing rings (28i, 28a) is axially supported by the fourth support surface (24) but not by the second support surface (32).

19. The device according to any one of the preceding claims, **characterised in that** the rotary bearing is a roller bearing which comprises two roller bearings (27, 28), and **in that** one of the roller bearings (27, 28) is arranged between the first support surface (31) and the third support surface (23), and the other of the roller bearings (27, 28) is arranged between the second support surface (32) and the fourth support surface (24), wherein the support surface (32) of the play reducing means (35) is formed such that, in the adjusting engagement of the play reducing means (35), it cannot be in contact either with the rotational member (20) or with the bearing body (30), but only with one of the roller bearings (27, 28).

20. The device according to the preceding claim, **characterised in that** the support surface (32) of the play reducing means (35) is formed such that, in the adjusting engagement of the play reducing means (35), it can only be in contact with one of the bearing rings of one of the roller bearings (27, 28).

21. The device according to any one of the preceding claims, **characterised in that** the play reducing means (35) is connected, in the adjusting position, by a material fit either to the bearing body (30) or to the rotational member (20), and thus secured.

22. The device according to any one of the preceding claims, **characterised in that** an adjusting member (35) which is axially rigid in its own right forms the play reducing means.

23. The device according to any one of the preceding claims, **characterised in that** the rotational member (20) forms the third support surface (23) and the fourth support surface (24) in one piece.

24. The device according to any one of the preceding claims, **characterised in that** the play reducing means (35) is in adjusting engagement with the bearing body (30).

25. The device according to any one of the preceding claims, **characterised in that** the adjusting engagement is based on a form fit and a force fit.

26. The device according to the preceding claim, **characterised in that** the adjusting engagement is a threaded engagement.

27. The device according to the preceding claim, **characterised in that** the bearing body (30) is a sleeve body which surrounds the rotational member (20) and comprises an inner thread (34) which is in threaded engagement with an outer thread of the play reducing means (35).

28. The device according to any one of the preceding claims, **characterised in that** the translational member (10) and the rotational member (20) each form at least one engaging flank (11f, 21f) and are in a flank engagement by means of the engaging flanks (11f, 21 f) such that the rotational movement of the rotational member (20) generates the translational movement of the translational member (10), and **in that** another play reducing means (25; 15, 17) is provided which, in an adjusting engagement with the translational member (10) and the rotational member (20), is displaced relative to the translational member (10) and the rotational member (20) into an adjusting position and is secured in the adjusting position such that an axial play in the flank engagement is reduced.

29. The device according to the preceding claim, **characterised in that** the flank engagement is a threaded engagement.

30. The device according to any one of the preceding two claims, **characterised in that** the adjusting engagement between the other play reducing means (25; 15, 17) and the translational member (10) is a threaded engagement.

31. The device according to any one of the preceding three claims, **characterised in that** the adjusting engagement between the other play reducing means (25) and the rotational member (20) is a threaded engagement.

## Revendications

1. Dispositif pour l'administration dosée d'un produit injectable, le dispositif comprenant:
a) un boîtier (1, 2), qui forme un réservoir de produit ou un logement pour un réservoir de produit (12),
b) un élément de translation (10), qui exécute un mouvement de translation dans une direction de translation (V) pour le transport du produit,
c) un élément de rotation (20), qui exécute pour le transport un mouvement de rotation autour d'un axe de rotation (T) et est couplé avec l'élément de translation (10) de telle façon qu'il supporte l'élément de translation (10) en sens inverse de la direction de translation et qu'un des mouvements de rotation ou de translation provoque l'autre mouvement,
d) un palier tournant, qui comprend un corps de palier (30; 1) formé par le boîtier (1, 2) ou soutenu axialement, qui supporte l'élément de rotation (20) de manière permettant la rotation autour de l'axe de rotation (T), et qui présente une première surface d'appui (31), une deuxième surface d'appui (32), une troisième surface d'appui (23) et une quatrième surface d'appui (24) pour sécuriser l'élément de rotation (20) axialement au corps de palier (30; 1),
**caractérisé en ce que**
e) la première surface d'appui (31) et la deuxième surface d'appui (32) sont reliées axialement de manière rigide au corps de palier (30; 1) et sont soit écartées axialement l'une de l'autre soit se font axialement face et la troisième surface d'appui (23) et la quatrième surface d'appui (24) font axialement face respectivement à la première surface d'appui (31) et à la deuxième surface d'appui (32) et sont reliées axialement de manière rigide à l'élément de rotation (20),
f) et **en ce que** le dispositif comprend un dispositif de réduction du jeu (35; 40, 50), qui presse axialement l'une contre l'autre avec une force de pressage au moins deux des surfaces d'appui (31, 32, 23, 24) qui se font face axialement et élimine ou réduit ainsi un jeu axial du palier tournant.

2. Dispositif suivant la revendication 1, **caractérisé en ce que** le dispositif de réduction du jeu (40, 50) génère la force de pressage en tant que force d'élasticité.

3. Dispositif suivant l'une des revendications précédentes, **caractérisé en ce que** le dispositif de réduction du jeu (40, 50) comprend un ressort (40) générant la force de pressage.

4. Dispositif suivant la revendication précédente, **caractérisé en ce que** le ressort (50) agit comme un ressort à lames.

5. Dispositif suivant l'une des deux revendications précédentes, **caractérisé en ce que** le ressort (50) est un corps annulaire axialement élastique entourant l'élément de rotation (20).

6. Dispositif suivant la revendication précédente, **caractérisé en ce que** le corps annulaire (50) est ondulé à la périphérie.

7. Dispositif suivant l'une des revendications précédentes, **caractérisé en ce que** le dispositif de réduction du jeu (40, 50) comprend un corps de transmission (40) mobile axialement relativement au boîtier (1) ou au corps de palier (30) qui est disposé entre deux (31, 23) des surfaces d'appui se faisant face axialement (31, 23, 32, 24) et transmet la force de pressage axiale entre ces surfaces d'appui (31, 23).

8. Dispositif suivant la revendication précédente, **caractérisé en ce que** le corps de transmission (40) est guidé axialement par le corps de palier (30) ou le boîtier (1) et en option également avec protection contre la torsion.

9. Dispositif suivant l'une des deux revendications précédentes, **caractérisé en ce que** les surfaces d'appui (31, 23) entre lesquelles le corps de transmission (40) est disposé sont décalées radialement l'une par rapport à l'autre et **en ce que** le corps de transmission (40) ponte ce décalage radial.

10. Dispositif suivant l'une des revendications précédentes, **caractérisé en ce qu'**une pièce de fond (37), de préférence un support de détecteur (37), du corps de palier (1) ou du boîtier (1) présente un socle dépassant axialement qui forme à son extrémité frontale la deuxième surface d'appui (32).

11. Dispositif suivant la revendication précédente, **caractérisé en ce que** l'élément de rotation (20) glisse en tournant sur le socle.

12. Dispositif suivant l'une des revendications précédentes, **caractérisé en ce que** l'élément de rotation (20) présente une nervure annulaire (22) qui forme au moins une des surfaces d'appui (23, 24) de l'élément de rotation (20).

13. Dispositif suivant l'une des revendications précédentes, **caractérisé en ce que** le dispositif de réduction du jeu (35) forme la première ou la deuxième surface d'appui (32) et est avec le corps de palier (30) dans une prise de réglage, dans laquelle la première surface d'appui (31) et la deuxième surface d'appui (32) sont réglées l'une par rapport à l'autre dans une position de réglage et fixées axialement l'une par rapport à l'autre dans la position de réglage de telle façon qu'un jeu axial du palier tournant soit réduit.

14. Dispositif suivant l'une des revendications précédentes, **caractérisé en ce que** le dispositif de réduction du jeu (35) forme la troisième ou la quatrième surface d'appui et est avec l'élément de rotation dans une prise de réglage, dans laquelle la troisième surface d'appui et la quatrième surface d'appui sont réglées l'une par rapport à l'autre dans une position de réglage et fixées axialement l'une par rapport à l'autre dans la position de réglage de telle façon qu'un jeu axial du palier tournant soit réduit.

15. Dispositif suivant l'une des revendications précédentes, **caractérisé en ce qu'**un palier à roulement (27) avec une bague de palier intérieure radiale (27i) et une bague de palier extérieure radiale (27a) est disposé entre la première surface d'appui (31) et la troisième surface d'appui (23).

16. Dispositif suivant la revendication précédente, **caractérisé en ce qu'**une des bagues de palier (27i, 27a) est supportée axialement par la première surface d'appui (31), mais pas par la troisième surface d'appui (23) et **en ce que** l'autre des bagues de palier (27i, 28a) est supportée axialement par la troisième surface d'appui (23), mais pas par la première surface d'appui (31).

17. Dispositif suivant l'une des revendications précédentes, **caractérisé en ce qu'**un palier à roulement (28) avec une bague de palier intérieure radiale (28i) et une bague de palier extérieure radiale (28a) est disposé entre la deuxième surface d'appui (32) et la quatrième surface d'appui (24).

18. Dispositif suivant la revendication précédente, **caractérisé en ce qu'**une des bagues de palier (28i, 28a) est supportée axialement par la deuxième surface d'appui (32), mais pas par la quatrième surface d'appui (24) et **en ce que** l'autre des bagues de palier (28i, 28a) est supportée axialement par la quatrième surface d'appui (24), mais pas par la deuxième surface d'appui (32).

19. Dispositif suivant l'une des revendications précédentes, **caractérisé en ce que** le palier tournant est un palier à roulement qui comprend deux roulements (27, 28) et **en ce qu'**un des roulements (27, 28) est disposé entre la première surface d'appui (31) et la troisième surface d'appui (23) et l'autre des roulements (27, 28) entre la deuxième surface d'appui (32) et la quatrième surface d'appui (24), la surface d'appui (32) du dispositif de réduction du jeu (35) étant formée de telle façon que, en prise de réglage avec le dispositif de réduction du jeu (35), elle ne puisse avoir de contact ni avec l'élément de rotation (20) ni avec le corps de palier (30), mais uniquement avec un des roulements (27, 28).

20. Dispositif suivant la revendication précédente, **caractérisé en ce que** la surface d'appui (32) du dispositif de réduction du jeu (35) est formée de telle façon que, dans la prise de réglage du dispositif de réduction du jeu (35), elle puisse uniquement être en contact avec une seule des bagues de palier d'un des roulements (27, 28).

21. Dispositif suivant l'une des revendications précédentes, **caractérisé en ce que** le dispositif de réduction du jeu (35) dans la position de réglage est relié par liaison de matière soit au corps de palier (30) soit à l'élément de rotation (20) et ainsi sécurisé.

22. Dispositif suivant l'une des revendications précédentes, **caractérisé en ce qu'**un élément de réglage axial rigide en soi (35) forme le dispositif de réduction du jeu.

23. Dispositif suivant l'une des revendications précédentes, **caractérisé en ce que** l'élément de rotation (20) forme en une pièce la troisième surface d'appui (23) et la quatrième surface d'appui (24).

24. Dispositif suivant l'une des revendications précédentes, **caractérisé en ce que** le dispositif de réduction du jeu (35) est en prise de réglage avec le corps de palier (30).

25. Dispositif suivant l'une des revendications précédentes, **caractérisé en ce que** la prise de réglage repose sur un blocage mécanique et un transfert de force.

26. Dispositif suivant la revendication précédente, **caractérisé en ce que** la prise de réglage est une coopération par filetage.

27. Dispositif suivant la revendication précédente, **caractérisé en ce que** le corps de palier (30) est un corps enveloppe, qui entoure l'élément de rotation (20) et présente un filet intérieur (34) qui est en coopération par filetage avec un filet extérieur du dispositif de réduction du jeu (35).

28. Dispositif suivant l'une des revendications précédentes, **caractérisé en ce que** l'élément de translation (10) et l'élément de rotation (20) forment chacun au moins un flanc d'attaque (11f, 21f) et sont à l'aide des flancs d'attaque (11f, 21f) dans une telle coopération de flancs que le mouvement de rotation de l'élément de rotation (20) provoque le mouvement de translation de l'élément de translation (10) et **en ce qu'**un dispositif supplémentaire de réduction du jeu (25; 15, 17) est prévu, qui, dans une prise de réglage avec l'élément de translation (10) et l'élément de rotation (20), est décalé relativement à l'élément de translation (10) et à l'élément de rotation (20) dans une position de réglage et fixé dans la position de réglage de telle façon qu'un jeu axial de la prise de flanc est réduit.

29. Dispositif suivant la revendication précédente, **caractérisé en ce que** la prise de flanc est une coopération par filetage.

30. Dispositif suivant l'une des deux revendications précédentes, **caractérisé en ce que** la prise de réglage du dispositif supplémentaire de réduction du jeu (25; 15, 17) avec l'élément de translation (10) est une coopération par filetage.

31. Dispositif suivant l'une des trois revendications précédentes, **caractérisé en ce que** la prise de réglage du dispositif supplémentaire de réduction du jeu (25) avec l'élément de rotation (20) est une coopération par filetage.
